# FASCICULE DE BREVET EUROPEEN

(11) **EP 2 834 253 B1**
(45) Date de publication et mention de la délivrance du brevet: **29.03.2017**
(21) Numéro de dépôt: 13713922.6
(22) Date de dépôt: 04.04.2013
(51) Int. Cl.: C07F 9/44

(54) **COMPOSÉS THIOL ET LEUR UTILISATION POUR LA SYNTHÈSE D'OLIGONUCLÉOTIDES MODIFIÉS**
THIOLVERBINDUNGEN UND VERWENDUNG DAVON ZUR HERSTELLUNG VON MODIFIZIERTEN OLIGONUKLEOTIDEN
THIOL COMPOUNDS AND THE USE THEREOF FOR THE SYNTHESIS OF MODIFIED OLIGONUCLEOTIDES

(30) Priorité: 04.04.2012 FR 1253121
(43) Date de publication de la demande: 11.02.2015
(73) Titulaire: Centre National de la Recherche Scientifique (C.N.R.S.), 75794 Paris Cedex 16 (FR); Universite de Montpellier 1, 34967 Montpellier Cedex 2 (FR); Etablissement Français du Sang, 93218 La Plaine Saint Denis Cedex (FR); Université Claude Bernard Lyon 1, 69622 Villeurbanne Cedex (FR)
(72) Inventeur: MORVAN, François, F-34170 Castelnau Le Lez (FR); MEYER, Albert, F-34470 Perols (FR); VASSEUR, Jean-Jacques, F-34980 Combaillaux (FR); MAYEN, Julie, 84270 Vedène (FR); CHAIX, Carole, F-69970 Chaponnay (FR); FARRE, Carole, F-38280 Villette d'Anthon (FR); FOURNIER-WIRTH, Chantal, F-93218 La Plaine Saint Denis Cedex (FR); CANTALOUBE, Jean-François, F-93218 La Plaine Saint Denis Cedex (FR); LEREAU, Myriam, F-93218 La Plaine Saint Denis Cedex (FR)
(74) Mandataire: Hirsch & Associés
(86) Numéro de dépôt international: PCT/EP2013/057122
(87) Numéro de publication internationale: WO 2013/150106

(56) Documents cités:
- UDDHAV KUMAR SHIGDEL ET AL: "A New 1'-Methylenedisulfide Deoxyribose that Forms an Efficient Cross-Link to DNA Cytosine-5 Methyltransferase (DNMT)", JOURNAL OF THE AMERICAN CHEMICAL SOCIETY, vol. 130, no. 52, 31 décembre 2008 (2008-12-31), pages 17634-17635, XP055045414, ISSN: 0002-7863, DOI: 10.1021/ja8064304
- SHENGXI JIN ET AL: "Synthesis of Amine- and Thiol-Modified Nucleoside Phosphoramidites for Site-Specific Introduction of Biophysical Probes into RNA", THE JOURNAL OF ORGANIC CHEMISTRY, vol. 70, no. 11, 1 mai 2005 (2005-05-01), pages 4284-4299, XP055045401, ISSN: 0022-3263, DOI: 10.1021/jo050061l
- HATANO A ET AL: "Synthesis and redox-active base-pairing properties of DNA incorporating mercapto C-nucleosides", TETRAHEDRON, ELSEVIER SCIENCE PUBLISHERS, AMSTERDAM, NL, vol. 61, no. 7, 14 février 2005 (2005-02-14), pages 1723-1730, XP004744667, ISSN: 0040-4020, DOI: 10.1016/J.TET.2004.12.038
- Z KUPIHÁR: "Synthesis and application of a novel, crystalline phosphoramidite monomer with Thiol Terminus, suitable for the synthesis of DNA conjugates", BIOORGANIC & MEDICINAL CHEMISTRY, vol. 9, no. 5, 1 mai 2001 (2001-05-01), pages 1241-1247, XP055045403, ISSN: 0968-0896, DOI: 10.1016/S0968-0896(00)00339-4

## Description

### DOMAINE TECHNIQUE

La présente invention concerne un composé thiol apte à former une chaîne d'oligomères pouvant être greffée sur un oligonucléotide. L'invention concerne également un oligonucléotide greffé par un tel composé, possédant ainsi une ou plusieurs fonctions thiol, apte à être immobilisé sur une surface d'or ou sur une surface greffée, notamment une surface greffée par des groupements maléimide ou acrylamide. L'invention concerne également un kit de test de détection d'interaction entre des nucléotides et d'autres molécules utilisant comme surface la surface d'or ou la surface greffée, notamment par des groupements maléimide ou acrylamide sur laquelle sont immobilisés les oligonucléotides selon l'invention.

### ETAT DE LA TECHNIQUE

Les oligonucléotides sont des molécules comprenant une courte chaîne de nucléotides, le nombre de nucléotides pouvant varier de 1 à environ 100. Ce sont des fragments d'ARN (acide ribonucléique) ou d'ADN (acide désoxyribonucléique). Les oligonucléotides sont synthétisés généralement sous forme de simples brins.

Les oligonucléotides peuvent être synthétisés par voie enzymatique ou par des méthodes de synthèse chimique. Lorsque l'on choisit une méthode chimique, habituellement, les oligonucléotides sont synthétisés sur support solide par des procédés bien connus de l'homme du métier, par exemple en mettant en oeuvre un procédé utilisant des phosphoramidites, des H-phosphonates, des phosphodiesters ou des phosphotriesters, les deux derniers composés étant des dérivés de l'acide phosphorique, tandis que les phosphoramidites sont des dérivés de l'acide phosphoreux.

Une propriété importante des oligonucléotides est leur capacité à contrôler les gènes et la fonction des protéines dans une séquence spécifique. Cette propriété fait des oligonucléotides et de leurs dérivés des composés très utilisés dans la recherche en biologie moléculaire et dans de nombreuses applications pharmaceutiques et de diagnostic. Le domaine de la recherche nécessite d'avoir à disposition de grandes quantités d'oligonucléotides. Ainsi, un procédé permettant de produire de grandes quantités d'oligonucléotides ayant une très haute pureté est nécessaire.

Les acides nucléiques ayant une séquence donnée de nucléotides sont largement utilisés pour la détection d'ADN dans des échantillons biologiques. Le principe de ce test de détection repose sur la complémentarité de la séquence de nucléotides avec la séquence cible, formant ainsi un double brin. Ce double brin est formé au cours d'un procédé nommé hybridation.

Ce phénomène d'hybridation peut ensuite être détecté par diverses méthodes, notamment en marquant la séquence de nucléotides destinée à se lier à la séquence initialement présente. Les méthodes de marquage, fluorescent ou radioactif, pour la détection du phénomène d'hybridation présentent certains inconvénients. Le marquage nécessite une étape supplémentaire lors de la synthèse de la séquence de nucléotides, cette étape étant parfois difficile à mettre en oeuvre. Le test basé sur ce phénomène d'hybridation détecté par marquage nécessite, après l'étape d'hybridation, d'éliminer les séquences de nucléotides marquées ne s'étant pas liées à la séquence cible de l'échantillon.

D'autres méthodes de détection ont alors été mises en oeuvre, notamment des méthodes dans lesquelles les séquences de nucléotides sont préalablement immobilisées sur une surface, l'échantillon à tester étant ensuite mis en contact avec cette surface afin de créer le phénomène d'hybridation par la formation de doubles brins.

Parmi ces autres méthodes, les méthodes électrochimiques sont de plus en plus utilisées. Ces méthodes sont basées sur l'immobilisation d'oligonucléotides sur une électrode, par exemple une électrode d'or. Au cours du test, l'hybridation des oligonucléotides simples brins immobilisés sur l'électrode d'or est quantifiée grâce à la mesure du courant électrique, qui dépend du nombre de doubles brins formés entre l'oligonucléotide simple brin immobilisé et l'oligonucléotide de l'échantillon.

Afin de réaliser ces tests, l'oligonucléotide est immobilisé sur la surface métallique, et notamment sur une surface d'or. Cette immobilisation peut notamment être obtenue grâce à une liaison entre un atome d'or et un atome de soufre. Cependant, la liaison Au-S est modérément forte. Ainsi, une seule liaison or-soufre ne suffit pas à immobiliser un oligonucléotide sur la surface d'or de façon très stable. En effet, les procédés de test supposent des étapes, notamment de lavage, qui occasionnent de fortes contraintes mécaniques susceptibles de déstabiliser la liaison Au-S.

Le document EP 0 523 978 divulgue des composés phosphoramidite ou phosphonate utilisables pour la production d'oligonucléotides modifiés par des thiols. Ils ne peuvent être introduits qu'une seule fois et uniquement sur l'extrémité 5' d'un oligonucléotide.

Le document US 7,601,848 divulgue un composé polyfonctionnel comprenant deux atomes de soufre destiné à être incorporé dans des oligomères afin de créer au moins deux liaisons or-soufre et ainsi stabiliser l'oligonucléotide sur la surface d'or. Certains de ces composés comportent une fonction phosphoramidite. Les composés utilisés dans ce document sont fabriqués à partir de composés très coûteux et le couplage du composé polyfonctionnel sur les oligonucléotides n'a pas un rendement satisfaisant en raison de l'encombrement stérique de ce composé. Dans ce document, un agent de liaison est nécessaire afin de lier les composés thiol entre eux et ainsi effectuer une multiple introduction de composés thiol dans un oligonucléotide.

U. K. Shigdel et C. He, J. Am. Chem. Soc. 2008, 130(52), 17634-5, décrivent un nucléotide modifié par une fonction phosphoramidite destiné à être introduit sur un oligonucléotide par cette fonction phosphoramidite. La synthèse du composé décrit est effectuée en 12 étapes.

S. Jin et al., J. Org. Chem. 2005, 70, 4284-4299, décrivent un nucléoside modifié par une fonction phosphoramidite et une fonction thiol. Les nucléosides modifiés sont utilisés pour introduire des sondes biophysiques au sein d'ARN. Le nucléoside modifié est synthétisé en 9 étapes avec un rendement global de 12,5%.

A. Hatano et al., Tetrahedron, 61, 2005, 1723-1730, décrivent la synthèse d'ADN faisant intervenir une base nucléoside comprenant une fonction phosphoramidite et une fonction thiophénol. Le nucléoside modifié est synthétisé en 7 étapes avec un rendement insuffisant, inférieur à 5%.

Dans les trois publications scientifiques ci-dessus, le composé phosphoramidite est un nucléoside modifié destiné à être incorporé dans un oligonucléotide par l'intermédiaire de cette fonction phosphoramidite. Les réactifs de départ sont chers, les synthèses sont longues et complexes et les rendements de synthèse de ces composés phosphoramidite sont faibles. De plus, l'encombrement stérique de ces composés osidiques n'est pas propice à un greffage satisfaisant sur une surface.
A. A. Rowe et al., Anal. Chem. 2011, 83, 9462-9466 décrivent un composé nucléosidique pouvant être immobilisé sur une surface d'or pour la détection d'ADN. Le composé nucléosidique est incorporé une seule fois, pour une immobilisation sur une surface d'or par l'intermédiaire d'une unique fonction thiol. Le réactif de départ est cher, et le composé thiol est complexe à fabriquer.

Le but de la présente invention est de fournir un composé thiol palliant au moins partiellement les inconvénients précités, c'est-à-dire un composé thiol, facile à fabriquer, pouvant être oligomérisé de manière simple et économique, et destiné à être incorporé dans un oligonucléotide et immobilisé sur une surface.

Plus particulièrement, l'invention vise à fournir une méthode permettant d'introduire facilement et efficacement une ou plusieurs fonctions thiol dans un oligonucléotide.

### RESUME DE L'INVENTION

Un premier objet de l'invention concerne un composé répondant à la formule (I) suivante : 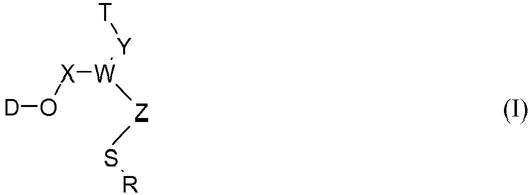 dans laquelle :
T est un groupement choisi parmi -O-P(OR₁)N(R₂)₂, -O-PH(O)O⁻, -OC(O)JC(O)NH-□,
   ∘ R₁ est choisi parmi les groupements 2-cyanoéthyle, R'₁R'₂R'₃SiCH₂CH₂, et R'₁, R'₂, R'₃ identiques ou différents représentent un groupement choisi parmi les alkyles linéaires ou ramifiés comportant de 1 à 12 atomes de carbone et les aryles en C6-C12,
   ∘ R₂ est choisi parmi les groupements alkyles linéaires ou ramifiés comportant de 1 à 12 atomes de carbone, pyrrolidine,
   ∘ J est choisi parmi une simple liaison, un groupement -CH₂-, -CH₂CH₂-, -CH₂OCH₂-, -CH₂OPhOCH₂- où Ph est un benzyle,
   ∘ □ représente un support solide,
D est un groupement protecteur des alcools choisi parmi le 4.4'-diméthoxytrityle, le 9-phenylxanthèn-9-yl, le fluorenylmethyloxycarbonyl et le tert-butyl-dméthylsilyl,
W est choisi parmi les groupements CH, CCH₃, CCH₂CH₃, cyclohexanes triyle, et benzènes triyle,
Z est choisi parmi les groupements alkoxy en C1-C12, cyclohétéroalkyles oxygéné ou azoté en C3-C12, NCO-alkyles en C1-C12, CON-alkyles en C1-C12,
Y est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
X est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
R est choisi parmi les groupements acyles en C1-C12, S-alkyles en C1-C12, S-aryles en C6-C12, S-2-pyridine, S-hétéroalkyles oxygénés ou azotés en C1-C12, S-cycloalkyles en C3-C12, S-cyclohétéroalkyles oxygénés ou azotés en C3-C12.

Selon un mode de réalisation, le composé répond à l'une des formules (Ia), (Ib) et (Ic) ci-dessous : 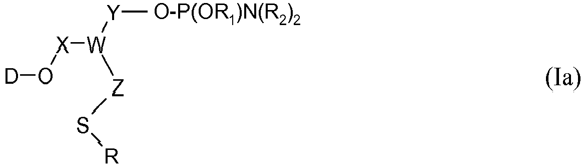 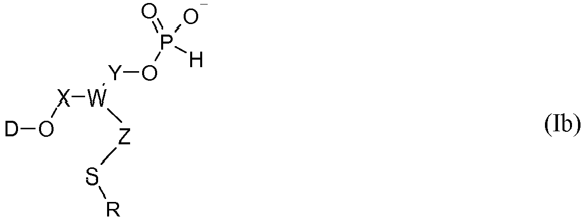 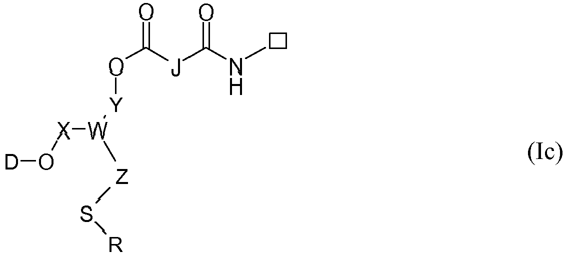 Selon un mode de réalisation,
- D est choisi parmi le 4,4'-diméthoxytrityle, le 9-phenylxanthen-9-yl ou le Fluorenylmethyloxycarbonyl,
- W est choisi parmi un groupement CH, CCH₃, CCH₂CH₃, un cyclohexane tri-yle et benzène tri-yle ; et/ou
- Z est choisi parmi les groupements alkoxy en C1-C6, cyclohétéroalkyles oxygéné ou azoté en C3-C6, NCO-alkyles en C1-C6, CON-alkyles en C1-C6 ; et/ou
- Y est choisi parmi les groupements alkyles linéaires ou ramifiés en C1-C6, aminoalkyles en C1-C6, alkoxy en C1-C6, cycloalkyles en C3-C6, cyclohétéroalkyles oxygénés ou azotés en C3-C6 ; et/ou
- X est choisi parmi les groupements alkyles linéaires ou ramifiés en C1-C6, aminoalkyles en C1-C6, alkoxy en C1-C6, cycloalkyles en C3-C6, cyclohétéroalkyles oxygénés ou azotés en C3-C6 ; et/ou
- R est choisi parmi les groupements acyles en C1-C6, S-alkyles en C1-C6, S-aryles en C6-C6, S-hétéroalkyles oxygénés ou azotés en C1-C6, S-cycloalkyles en C3-C6, S-cyclohétéroalkyles oxygénés ou azotés en C3-C6, de préférence R est un acyle en C1-C6.

Selon un mode de réalisation, le groupement T est un groupement -O-P(OR₁)N(R₂)₂ où R₂ est un groupement isopropyle et R₁ est choisi parmi les groupements 2-cyanoéthyle, 2-(triméthylsilyl)éthyle, 2-(triphénylsilyl)éthyle, 2-(diphénylméthylsilyl)éthyle.

Selon un mode de réalisation, le groupement T est le groupement - OC(O)JC(O)NH-□ où □ est un support solide choisi parmi les résines, en particulier parmi les résines à base de polystyrène, polyacrylamide, polyéthylèneglycol, cellulose, polyéthylène, polyester, latex, polyamide, polydiméthylacrylamide, les polymères hydrophiles synthétiques ou naturels, les billes de verre, les gels de silice.

Un autre objet de l'invention concerne un procédé de fabrication d'un composé de formule (I) selon l'invention comprenant au moins une étape choisie parmi les étapes suivantes :
- préparation du composé (Ia) à partir du composé (II) selon le schéma de synthèse suivant : 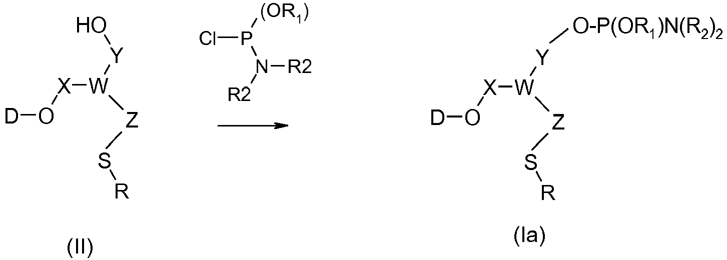 ou selon le schéma de synthèse suivant : 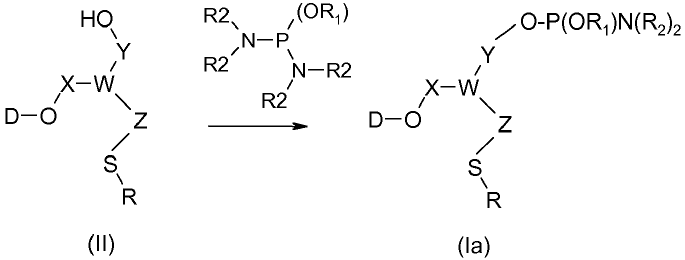
- préparation du composé (Ib) à partir du composé (II) selon le schéma de synthèse suivant : 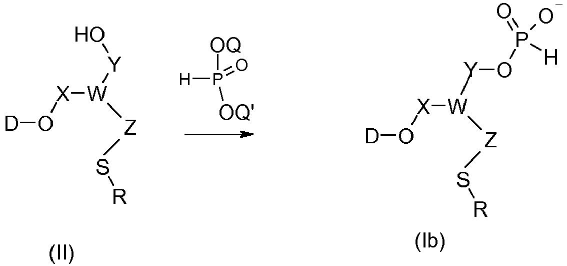 dans laquelle Q et Q' représentent, indépendamment l'un de l'autre, un groupement benzène substitué ou non,
- préparation du composé (Ic) à partir du composé (II) selon le schéma de synthèse suivant : 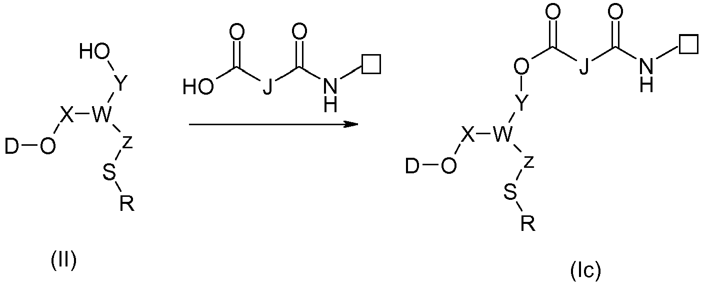 ou selon le schéma de synthèse suivant : 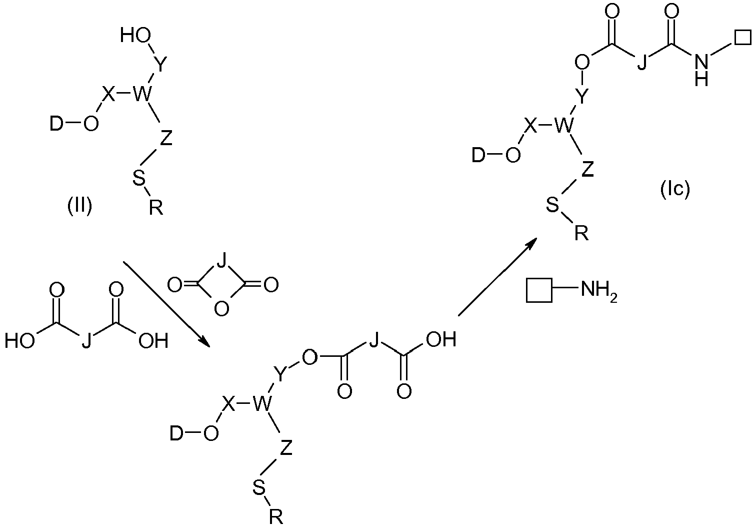 D, X, Y, Z, W, R, □, R₁ et R₂ ayant la même définition dans chacune de ces étapes que dans le composé (I).

Un autre objet de l'invention concerne un oligomère susceptible d'être obtenu par oligomérisation d'un composé (I) selon l'invention, répondant à la formule

(Ic) - (Δ)ₖ (XVI)ₖ

dans laquelle :
(Ic) a la même signification que ci-dessus, le groupement R du composé (Ic) peut en outre représenter H.
k représente un entier compris entre 1 et 12,
Δ représente (I'a) ou (I'b) où 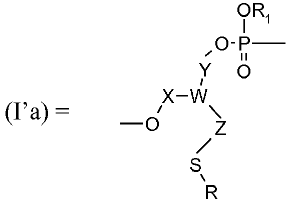 et 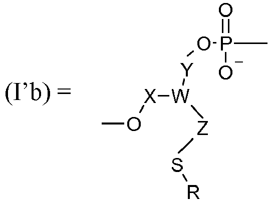 dans lesquelles, X, Y, W, Z, R, R₁ ont la même définition que pour le composé (I), R peut en outre représenter H.

Selon un mode de réalisation, l'oligomère selon l'invention répond à la formule (XIV)ₖ : 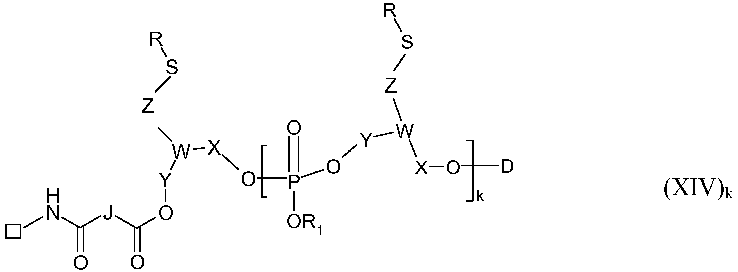 dans laquelle,
□, D, X, Y, W, Z, R et R₁ ont la même définition que pour le composé (I), R peut en outre représenter H et D peut en outre représenter H,
k est un nombre entier compris entre 1 et 11.

L'invention concerne également un procédé de préparation d'un oligonucléotide modifié comprenant au moins :
- une étape de greffage d'un composé (I) selon l'invention sur un oligonucléotide, ou
- une étape de greffage d'un nucléotide sur un oligomère selon l'invention.

Un autre objet de l'invention concerne un oligonucléotide modifié susceptible d'être obtenu par le procédé selon l'invention répondant à la formule (XIIa) suivante :

□ N₁ - N₂- ...- Nₙ₋₁- Nₙ- (I')_{y} -(M₁ -...- Mₘ₋₁- Mₘ)ₚ - (I')_{y'}

dans laquelle,
N₁, ...Nₙ représentent indépendamment les uns des autres un nucléotide,
M₁, ...Mₘ représentent indépendamment les uns des autres un nucléotide,
(I') représente un composé de formule (I'a) ou (I'b) tel que définis ci-dessus,
n est un nombre entier compris entre 1 et 100,
m est un nombre entier compris entre 1 et 100,
y est un nombre entier compris entre 1 et 12,
p représente 0 ou 1,
y' est un nombre entier compris entre 0 et 12 si p vaut 1 et, y' est égal à 0 si p vaut 0,
□ représente un support solide.

Un autre objet de l'invention concerne un oligonucléotide modifié susceptible d'être obtenu par le procédé selon l'invention répondant à la formule (XIIIa) suivante :

(Ic) - (I')_{y-1} - N₁ - N₂- ...- Nₙ₋₁- Nₙ - (I')_{y'} -[M₁ - M₂ - ... - Mₘ₋₁ - Mₘ]ₚ-(I')_{y"}

dans laquelle,
N₁, ...Nₙ représentent indépendamment les uns des autres un nucléotide,
M₁, ... Mₘ représentent indépendamment les uns des autres un nucléotide,
(I') représente un composé de formule (I'a) ou (I'b) tel que définis ci-dessus,
n est un nombre entier compris entre 1 et 100,
m est un nombre entier compris entre 1 et 100,
y est un nombre entier compris entre 1 et 12,
y' est un nombre entier compris entre 0 et 12,
p vaut 0 ou 1 si y' est différent de 0 et, si y' vaut 0 alors p vaut 0,
y" est un nombre entier compris entre 0 et 12 si p vaut 1 et, si p vaut 0 alors y" vaut 0.

Selon un mode de réalisation, l'oligonucléotide modifié selon l'invention répond à la formule (XIIc) : 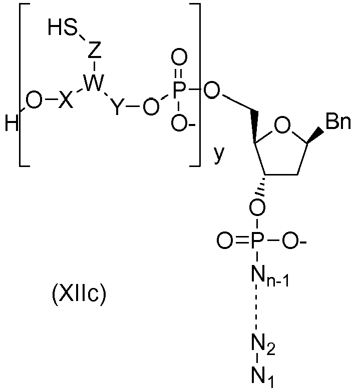 dans laquelle,
n, y, N₁, ...Nₙ₋₁ ont la même définition que ci-dessus,
X, Y, Z, W ont la même définition que ci-dessus,
Bn représente la base du n-ième nucléotide.

Selon un mode de réalisation, l'oligonucléotide modifié selon l'invention répond à la formule (XIIIc) : 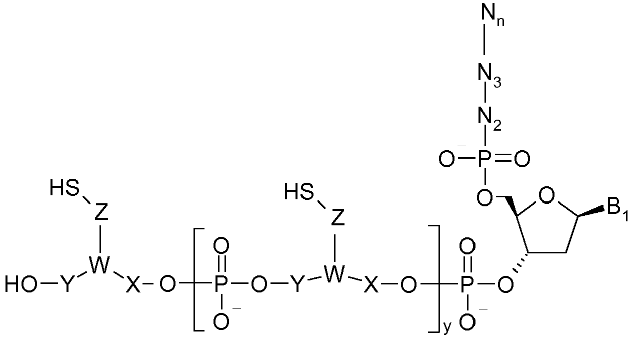 dans laquelle,
n, y, N ont la même définition que ci-dessus,
X, Y, Z, W ont la même définition que ci-dessus,
B₁ représente la base du 1er nucléotide.

Un autre objet de l'invention concerne un dispositif automatisé pour la synthèse de nucléotides comportant au moins des réservoirs distincts contenant :
- des nucléotides,
- des activateurs de couplage et
- des produits de lavage,
des moyens mécaniques de prélèvement et de distribution d'échantillons de produits, ainsi que des moyens informatiques permettant la mise en oeuvre contrôlée de ces moyens mécaniques, ainsi que :
au moins un réservoir dans lequel est placé un support solide greffé par un oligomère selon l'invention, et/ou au moins un réservoir contenant un composé (Ia) ou un composé (Ib) selon l'invention.

Un autre objet de l'invention concerne un substrat greffé par au moins un oligonucléotide modifié selon l'invention, ledit substrat comportant au moins une zone de réception revêtue d'un film d'or ou de platine ou greffée par des groupements comportant au moins une double liaison carbone-carbone ou une triple liaison carbone-carbone ou des groupements halogénoacétamide, de préférence des groupements maléimide et/ou acrylamide, ledit substrat étant en métal dans le cas du film d'or et en plastique dans le cas du greffage par des groupements comportant au moins une double liaison carbone-carbone ou une triple liaison carbone-carbone ou des groupements halogénoacétamide, de préférence des groupements maléimide et/ou acrylamide.

Selon un mode de réalisation de l'invention, les groupements comportant au moins une double liaison carbone-carbone ou une triple liaison carbone-carbone sont choisis parmi les alcènes activés par une fonction carbonyle en alpha, de préférence les alcènes sont choisis parmi les groupements maléimide, acrylamide.

Selon un mode de réalisation, les groupements halogénoacétamide sont choisis parmi les groupements bromoacétamido et iodoacétamido.

Selon un mode de réalisation de l'invention, les groupements comportant au moins une triple liaison carbone-carbone sont choisis parmi les alcynes activés par une fonction carbonyle en alpha, de préférence les alcynes sont choisis parmi les groupements 2-propynamide.

Selon un mode de réalisation, le substrat en métal est en cuivre ou en titane et/ou le substrat en plastique est en polystyrène.

L'invention propose également un kit de test comprenant :
- au moins un substrat comportant au moins une zone de réception revêtue d'un film métallique d'or ou de platine ou d'un substrat greffé par des groupements comportant au moins une double liaison carbone-carbone ou une triple liaison carbone-carbone ou des groupements halogénoacétamide, de préférence des groupements maléimide et/ou acrylamide,
- au moins un oligonucléotide modifié selon l'invention.

Un autre objet de l'invention concerne une utilisation d'un substrat selon l'invention pour réaliser des tests d'affinité entre un oligonucléotide et une autre molécule.

Les avantages de la présente invention sont les suivants :
- le composé thiol de l'invention est peu coûteux,
- le composé thiol de l'invention est obtenu par un procédé simple à mettre en oeuvre,
- le composé thiol de l'invention peut être introduit une ou plusieurs fois dans un oligonucléotide de manière simple et efficace,
- l'oligonucléotide greffé de l'invention peut être immobilisé de manière stable sur une surface d'or, ou sur une surface greffée, notamment avec des groupements maléimide ou acrylamide,
- les oligonucléotides de l'invention peuvent être fabriqués par un procédé totalement automatisable.

D'autres caractéristiques et avantages de l'invention apparaîtront à la lecture de la description qui suit d'un mode de réalisation préféré de l'invention, donnée à titre d'exemple et en référence aux dessins annexés.

### BREVE DESCRIPTION DES FIGURES

La figure 1 représente un schéma décrivant un procédé de synthèse des composés (I).
Les figures 2A et 2B représentent respectivement un schéma décrivant un procédé de synthèse d'un oligomère des composés de l'invention à partir de composés (Ia) et à partir de composés (Ib).
La figure 3 représente un schéma décrivant un procédé de synthèse d'un composé (XIIc) oligonucléotide greffé par un oligomère de (I) à son extrémité 5'.
La figure 4 représente un schéma exposant un procédé de synthèse d'un composé (XIIIc) oligonucléotide greffé par un oligomère de (I) à son extrémité 3'.
La figure 5 représente un histogramme du taux de greffage d'oligonucléotides modifiés sur une surface d'or.
La figure 6 représente un schéma exposant la stabilité du greffage d'oligonucléotides modifiés sur une surface d'or en fonction du temps à 60°C.
La figure 7 représente un schéma exposant la stabilité du greffage d'oligonucléotides modifiés sur une surface d'or en fonction du temps à 80°C.
La figure 8 représente un diagramme schématisant les résultats d'un test ELOSA avec détection par fluorescence.
La figure 9 représente les résultats d'un test d'hydridation sonde/cible avec une sonde monothiol.
La figure 10 représente les résultats d'un test d'hydridation sonde/cible avec une sonde dithiol.
La figure 11 représente les résultats d'un test d'hydridation sonde/cible avec une sonde tétrathiol.
La figure 12 représente les résultats d'un test d'hydridation sonde/cible avec une sonde tétrathiol greffée sur surface d'or.
La figure 13a représente les réactions entre l'oligonucléotide modifié selon l'invention et la surface greffée par des groupements alcényles ou alcynyles activés.
La figure 13b représente les réactions entre l'oligonucléotide modifié selon l'invention et la surface greffée par des groupements alcényles ou alcynyles avec activation lumineuse (λ = 265 nm).
La figure 14 représente les résultats d'un test d'hybridation sonde/cible avec des sondes présentant respectivement 1, 2, 4, 6 et 8 composés thiol selon l'invention.

### EXPOSE DES MODES DE REALISATION DE L'INVENTION

La présente invention concerne la préparation et l'utilisation de composés de structure phosphoramidite, H-phosphonate ou de composés reliés à un support solide présentant une fonction thiol protégée. Ces composés thiol sont destinés à être introduits dans des oligonucléotides. Les oligonucléotides ainsi obtenus peuvent présenter plusieurs fonctions thiol.

### Composé thiol

Les composés de la présente invention répondent à la formule (I) suivante : 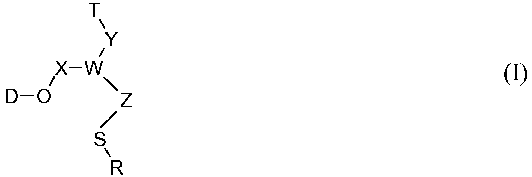 dans laquelle :
T est un groupement choisi parmi -O-P(OR₁)N(R₂)₂, -O-PH(O)O⁻, -OC(O)JC(O)NH-□,
   ∘ R₁ est choisi parmi les groupements 2-cyanoéthyle, R'₁R'₂R'₃SiCH₂CH₂, et R'₁, R'₂, R'₃, identiques ou différents, représentent un groupement choisi parmi les alkyles linéaires ou ramifiés comportant de 1 à 12 atomes de carbone et les aryles en C6-C12,
   ∘ R₂ est choisi parmi les groupements alkyles linéaires ou ramifiés comportant de 1 à 12 atomes de carbone, pyrrolidine
   ∘ J est choisi parmi une simple liaison, un groupement -CH₂-, -CH₂CH₂-, -CH₂OCH₂-, -CH₂OPhOCH₂- où Ph est un benzyle,
   ∘ □ représente un support solide,
D est un groupement protecteur des alcools choisi parmi le 4.4'-diméthoxytrityle, le 9-phenylxanthèn-9-yl, le fluorenylmethyloxycarbonyl et le tert-butyl-dméthylsilyl,
W est choisi parmi les groupements CH, CCH3, CCH2CH3, cyclohexanes triyle, et benzènes triyle,
Z est choisi parmi les groupements alkoxy en C1-C12, cyclohétéroalkyles oxygéné ou azoté en C3-C12, NCO-alkyles en C1-C12, CON-alkyles en C1-C12,
Y est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
X est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
R est choisi parmi les groupements acyles en C1-C12, S-alkyles en C1-C12, S-aryles en C6-C12, S-2-pyridine, S-hétéroalkyles oxygénés ou azotés en C1-C12, S-cycloalkyles en C3-C12, S-cyclohétéroalkyles oxygénés ou azotés en C3-C12.

Au sens de la présente invention, par « alcane tri-yle » on entend les alcanes triyle, linéaires, ramifiés ou cycliques, éventuellement substitués par un ou plusieurs groupements alkyles.

Parmi les groupements aryles tri-yles qui peuvent être présents dans le composé selon l'invention, on peut citer le benzène tri-yle, le naphtalène tri-yle.

Parmi les groupements aralcanes tri-yles qui peuvent être présents dans le composé selon l'invention, on peut citer le 1,3,5-triméthylbenzène tri-yle, le triméthylnaphtalène tri-yle.

Le composé (I) peut être distingué en trois sous-composés (Ia), (Ib) et (Ic) répondant aux formules (Ia), (Ib) et (Ic) ci-dessous dans lesquelles les paramètres X, Y, Z, R, R₁, R₂ et D ont la même définition qu'exposée ci-dessus pour la formule (I) :

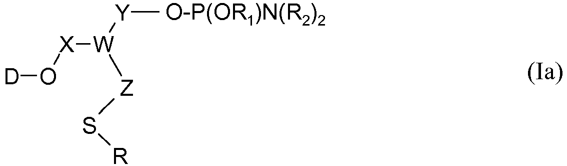

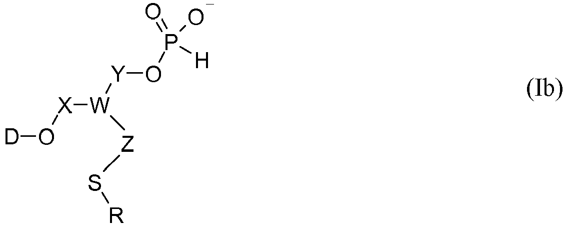

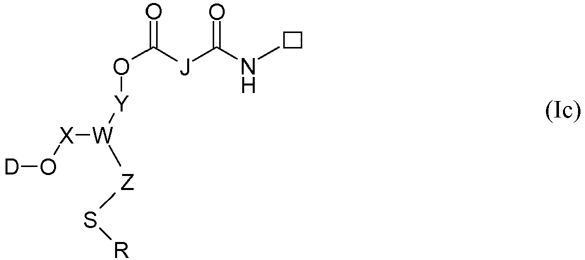

De préférence, R₁ est choisi parmi les groupements 2-cyanoéthyle et R'₁R'₂R'₃SiCH₂CH₂, et R'₁, R'₂, R'₃ identiques ou différents représentent un groupement choisi parmi les groupements alkyles linéaires ou ramifiés comportant de 1 à 6 atomes de carbone, le phényl ; préférentiellement R₁ est choisi parmi les groupements 2-cyanoéthyle et R'₁R'₂R'₃SiCH₂CH₂, et R'₁, R'₂, R'₃ identiques ou différents représentent un groupement choisi parmi les groupements alkyles linéaires ou ramifiés comportant de 1 à 3 atomes de carbone, le phényl ; encore plus préférentiellement R₁ est choisi parmi les groupements 2-cyanoéthyle, 2-(triméthylsilyl)éthyle, 2-(triphénylsilyl)éthyle, 2-(diphénylméthylsilyl)éthyle.

De préférence, R₂ est choisi parmi les groupements alkyles linéaires ou ramifiés comportant de 1 à 6 atomes de carbone. De préférence, R₂ est un groupement isopropyle (iPr).

De préférence, le support solide □ est choisi parmi les résines, en particulier parmi les résines à base de polystyrène, polyacrylamide, polyéthylène glycol, cellulose, polyéthylène, polyester, latex, polyamide, polydiméthylacrylamide, les polymères hydrophiles synthétiques ou naturels, les billes de verre, les gels de silice.

De préférence, W est choisi parmi les groupements CH, CCH₃, CCH₂CH₃, les cyclohexanes triyle, et les benzènes triyle.

De préférence, D est choisi parmi le 4,4'-diméthoxytrityle (DMTr), le 9-phenylxanthen-9-yl (pixyl) ou le Fluorenylmethyloxycarbonyl (Fmoc). Le groupement protecteur pixyl est décrit notamment dans le document Chattopadhyaya et Reese, Chem. Soc. Chem. Comm., 1978, 639-640. Un autre groupement protecteur des alcools peut être un groupement tert-butyl-diméthylsilyl, dans ce cas, un support en polystyrène sera particulièrement préféré.

De préférence, Z est choisi parmi les groupements aminoalkyles en C1-C6, alkoxy en C1-C6, cyclohétéroalkyles oxygéné ou azoté en C3-C6, NCO-alkyles en C1-C6, CON-alkyles en C1-C6.

De préférence, Y est choisi parmi les groupements alkyles linéaires ou ramifiés en C1-C6, aminoalkyles en C1-C6, alkoxy en C1-C6, cycloalkyles en C3-C6, cyclohétéroalkyles oxygénés ou azotés en C3-C6.

De préférence, X est choisi parmi les groupements alkyles linéaires ou ramifiés en C1-C6, aminoalkyles en C1-C6, alkoxy en C1-C6, cycloalkyles en C3-C6, cyclohétéroalkyles oxygénés ou azotés en C3-C6.

De préférence, R est choisi parmi les groupements acyles en C1-C12, S-alkyles en C1-C6, S-aryles en C6, S-hétéroalkyles oxygénés ou azotés en C6, S-cycloalkyles en C6, S-cyclohétéroalkyles oxygénés ou azotés en C6.

Selon un mode de réalisation, les alkyles linéaires ou ramifiés sont choisis parmi les groupements méthyle, éthyle, propyle, butyle, pentyle, hexyle, heptyle, octyle, nonyle, décyle, undécyle, dodécyle, isopropyle, isobutyle, *tert*-butyle.

Selon un mode de réalisation, les aminoalkyles sont choisis parmi les groupements aminométhyle, aminoéthyle, aminopropyle, aminobutyle, aminopentyle, aminohexyle, aminoheptyle, aminooctyle, aminononyle, aminodécyle, aminoundécyle, aminododécyle, aminoisopropyle, aminoisobutyle, amino-*tert*-butyle comprenant un ou plusieurs atomes d'azote.

Selon un mode de réalisation, les alkoxy sont choisis parmi les groupements méthoxy, éthoxy, propyloxy, oxybutyloxy, pentyloxy, hexyloxy, heptyloxy, octyloxy, nonyloxy, décyloxy, undécyloxy, dodécyloxy, isopropyloxy, isobutyloxy, *tert-*butyloxy comprenant un ou plusieurs atomes d'oxygène.

Selon un mode de réalisation, les cycloalkyles sont choisis parmi les cycles, comprenant éventuellement une ou plusieurs insaturations, comprenant entre 3 et 12 atomes de carbone, de préférence 6 atomes de carbone.

Selon un mode de réalisation, les cyclohétéroalkyles sont choisis parmi les cycles substitués par un ou plusieurs atomes d'azote et/ou d'oxygène, comprenant éventuellement une ou plusieurs insaturations et comprenant entre 3 et 12 atomes de carbone, de préférence 5 atomes de carbone et un atome d'azote ou d'oxygène.

Selon un mode de réalisation, les NCO-alkyles et CON-alkyles sont des groupements dans lesquels les alkyles peuvent être des alkyles linéaires ou ramifiés choisis parmi les groupements méthyle, éthyle, propyle, butyle, pentyle, hexyle, heptyle, octyle, nonyle, décyle, undécyle, dodécyle, isopropyle, isobutyle, *tert*-butyle.

Selon un mode de réalisation, R₂ est un groupement isopropyle (iPr) et/ou R₁ est un groupement cyanoéthyle.

Selon un mode de réalisation préféré, le composé thiol (Ia) est le composé (VI) répondant à la formule suivante : 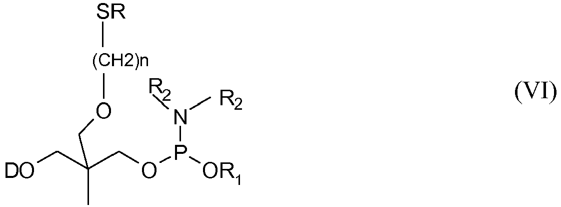 dans laquelle,
n est un nombre entier compris entre 1 et 12, de préférence compris entre 1 et 6,
R, R₁, R₂ et D ont la même définition que ci-dessus pour (Ia).

De préférence, R₂ est un groupement isopropyle (iPr) et R₁ est un groupement cyanoéthyle.

De préférence, R est un groupement acétyle.

De préférence, D est le 4,4'-diméthoxytrityle.

Selon un autre mode de réalisation, le composé thiol (Ia) est le composé (VII) répondant à la formule suivante : 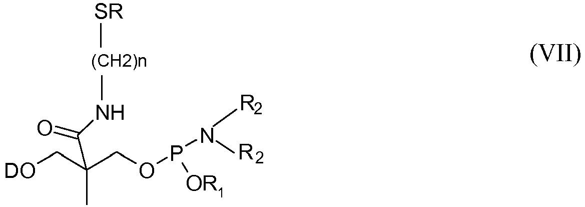 dans laquelle,
n est un nombre entier compris entre 1 et 12, de préférence compris entre 1 et 6,
R, R₁, R₂ et D ont la même définition que ci-dessus pour (Ia).

De préférence, R₂ est un groupement isopropyle (iPr) et R₁ est un groupement cyanoéthyle.

De préférence, R est un groupement acétyle.

De préférence, D est le 4,4'-diméthoxytrityle.

Selon un mode de réalisation, le composé thiol (Ic) est le composé (VIII) répondant à la formule suivante : 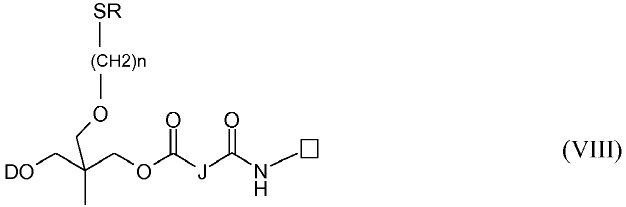 dans laquelle,
n est un nombre entier compris entre 1 et 12, de préférence entre 1 et 6.
R, D et □ ont la même définition que ci-dessus pour (Ic).

De préférence, R est un groupement acétyle. De préférence, J est un groupement éthyle. De préférence, D est le 4,4'-diméthoxytrityle.

Selon un mode de réalisation, le composé thiol (Ia) est le composé (IX) de formule : 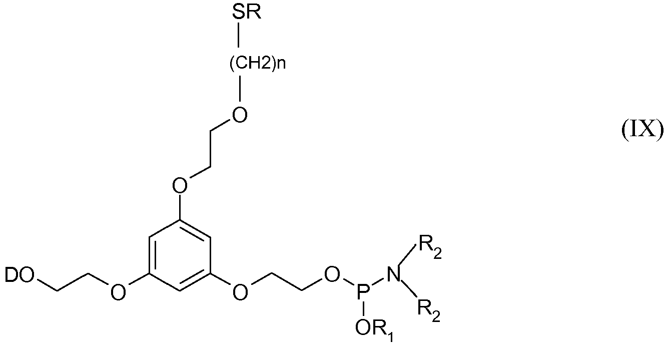 dans laquelle,
n est un nombre entier compris entre 1 et 12, de préférence entre 1 et 6,
R, R₁, R₂ et D ont la même définition que ci-dessus pour (Ia).

De préférence, R₂ est un groupement isopropyle (iPr) et R₁ est un groupement cyanoéthyle.

De préférence, R est un groupement acétyle.

De préférence, D est le 4,4'-diméthoxytrityle.

Selon un mode de réalisation, le composé thiol (Ia) est le composé (X) de formule : 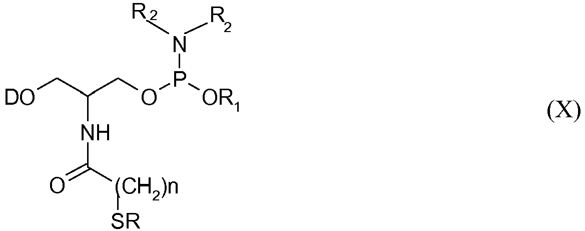 dans laquelle,
n est un nombre entier compris entre 1 et 12, de préférence entre 1 et 6,
R, R₁, R₂ et D ont la même définition que ci-dessus pour (Ia).

De préférence, R₂ est un groupement isopropyle (iPr) et R₁ est un groupement cyanoéthyle.

De préférence, R est un groupement acétyle.

De préférence, D est le 4,4'-diméthoxytrityle.

De préférence, R₂ est un groupement isopropyle (iPr) et R₁ est un groupement cyanoéthyle.

Selon un mode de réalisation, le composé thiol (Ia) est le composé (XI) de formule : 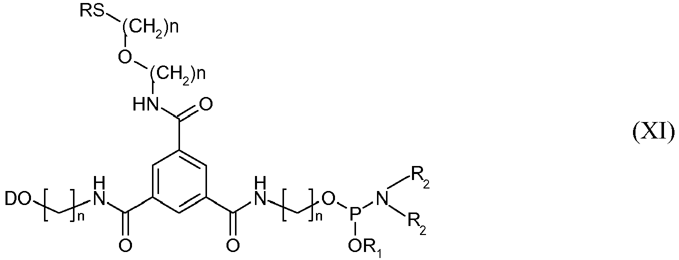 dans laquelle,
n est un nombre entier compris entre 1 et 12, de préférence compris entre 1 et 6,
R, R₁, R₂ et D ont la même définition que ci-dessus pour (Ia).

De préférence, R₂ est un groupement isopropyle (iPr) et R₁ est un groupement cyanoéthyle.

De préférence, R est un groupement acétyle.

De préférence, D est le 4,4'-diméthoxytrityle.

### Procédé de fabrication

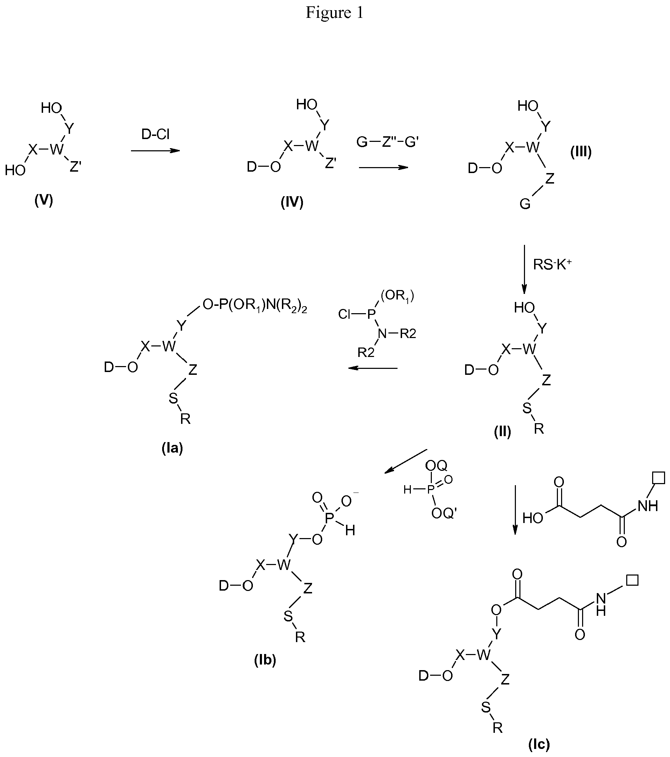

Le procédé de fabrication des composés (Ia), (Ib) et (Ic) est représenté sur le schéma de la figure 1.

Les composés (Ia), (Ib) et (Ic) sont obtenus à partir du même composé (II) présentant la formule suivante : 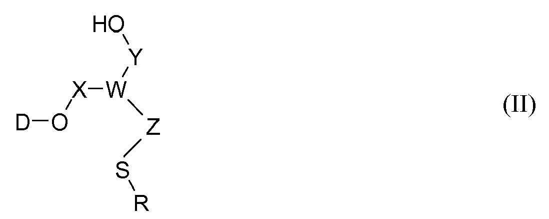 dans laquelle, D, X, W, Y, Z et R ont la même définition que dans le composé thiol (I).

Le composé de formule (Ia) peut être obtenu selon la réaction représentée sur le schéma suivant : 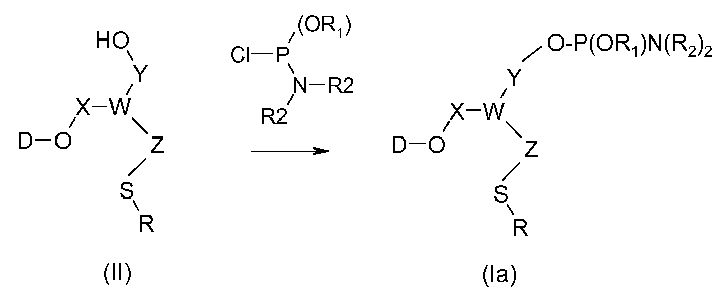 ou selon la réaction représentée sur le schéma suivant, de préférence en présence du sel de diisopropylamine tétrazolure :

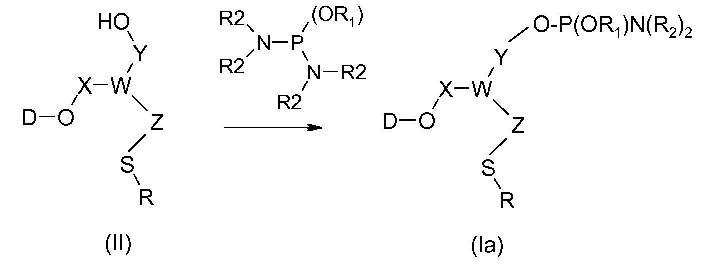

Le composé de formule (Ib) peut être obtenu selon la réaction représentée sur le schéma suivant : 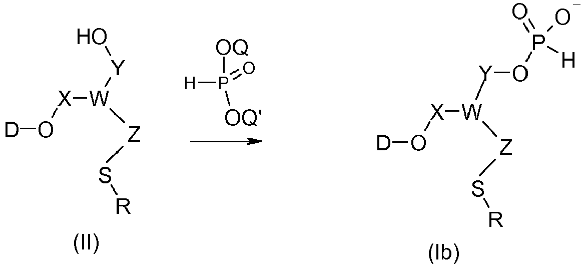 dans laquelle, Q et Q' représentent, indépendamment l'un de l'autre, un groupement benzène substitué ou non.

La réaction précédente d'obtention du composé (Ia) ou (Ib) s'effectue à partir du composé (II), de préférence en présence d'une base, par exemple la diisopropyléthylamine (DIEA), dans un solvant anhydre, tel que le dichlorométhane anhydre.

Le composé de formule (Ic) est également obtenu à partir du composé (II) mais de préférence selon l'étape de réaction représentée sur le schéma suivant :

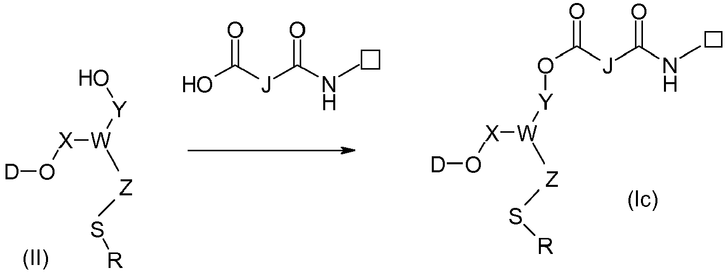

La réaction précédente d'obtention du composé (Ic) s'effectue de préférence dans un solvant anhydre, tel que la pyridine, en présence d'une base, telle que la triéthylamine.

On peut également obtenir le composé (Ic) selon le schéma réactionnel suivant :

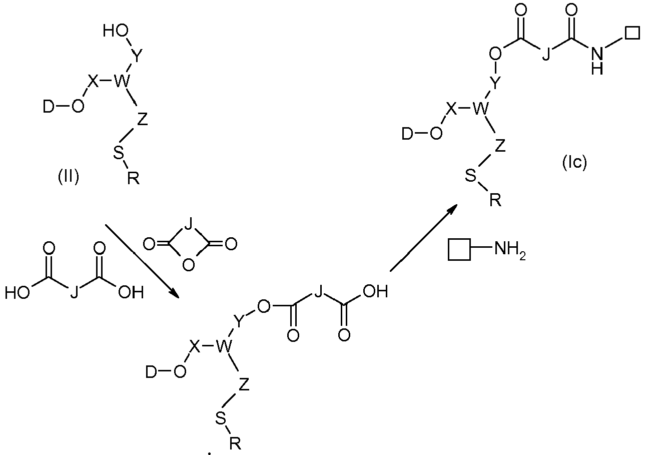

Un objet de la présente invention est le composé (II) de formule ci-dessus dans laquelle les groupements D, X, W, Z, Y et R ont la même définition que dans le composé (I).

Le composé de formule (II) peut être obtenu à partir du composé (III) selon l'étape de réaction décrite ci-dessous : 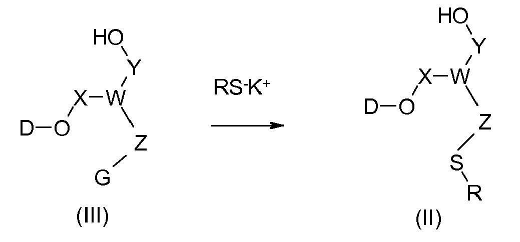 dans laquelle G est un halogène, de préférence le brome ou l'iode.

La réaction décrite ci-dessus s'effectue de préférence dans un solvant anhydre, tel que le toluène anhydre et en présence d'un éther couronne.

Le composé (III) peut être obtenu à partir du composé (IV) selon l'étape de réaction décrite ci-dessous : 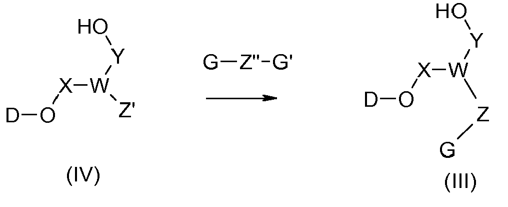 dans laquelle,
G et G' sont des atomes d'halogène identiques ou différents, de préférence G et G' sont des atomes de brome ou d'iode,
Z' est un groupement aminoalkyle en C1-C12, alkoxy en C1-C12, cyclohétéroalkyle oxygéné ou azoté en C3-C12, NCO-alkyle en C1-C12, CON-alkyle en C1-C12,
Z" est un groupement alkyle linéaire ou ramifié en C1-C12, aminoalkyle en C1-C12, alkoxy en C1-C12, cycloalkyle en C3-C12, cyclohétéroalkyle oxygéné ou azoté en C3-C12, NCO-alkyle en C1-C12, CON-alkyle en C1-C12,
le composé dihalogéné G-Z"-G' étant destiné à réagir avec le groupement Z' du compose (IV) pour conduire à la formation du groupement Z-G du composé (III).

L'étape d'obtention du composé (III) s'effectue de préférence en présence d'un hydrure alcalin, tel que NaH.

Le composé (IV) peut être obtenu à partir du composé commercial (V) par protection de la fonction alcool, selon l'étape de réaction suivante :

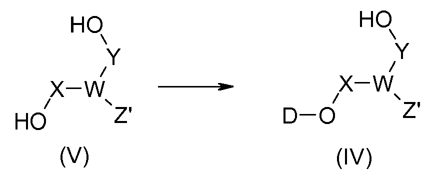

Cette étape de protection de la fonction alcool est effectuée dans des conditions bien connues de l'homme du métier, en fonction du choix de D.

Selon un mode de réalisation, le composé (IV) est obtenu à partir du composé (V) par réaction avec le 4,4'-chlorure de diméthoxytrityle (DMTr-Cl) de préférence dans un solvant, tel que la pyridine afin de protéger la fonction alcool.

Selon un autre mode de réalisation, le composé (IV) est obtenu à partir du composé (V) à partir du chlorure de 9-phénylxantèn-9-yle (pixyl-Cl) dans les conditions décrites dans le document Chattopadhyaya et Reese, Chem. Soc. Chem. Comm., 1978, 639-640.

Selon un autre mode de réalisation, le composé (IV) est obtenu à partir du composé (V) par réaction avec le chlorure de fluorenylmethyloxycarbonyle (Fmoc-Cl) dans des conditions bien connues de l'homme du métier.

Dans les formules précédentes (II) à (V), X, Y, W, Z, D, R, R₁, R₂ ont les mêmes définitions que dans la définition du composé (I) indiquée ci-dessus.

De préférence, le composé (V) de départ est le 1,1,1-tris(hydroxyméthyl)éthane ou l'acide 2,2-bis(hydroxyméthyl) propionique ou le 1,3,5-tris(hydroxyethoxy)benzène ou le 1,3,5-tris(hydroxyméthyl)cyclohexane ou le 2-amino-1,3-propanediol.

### Oligomère du composé thiol

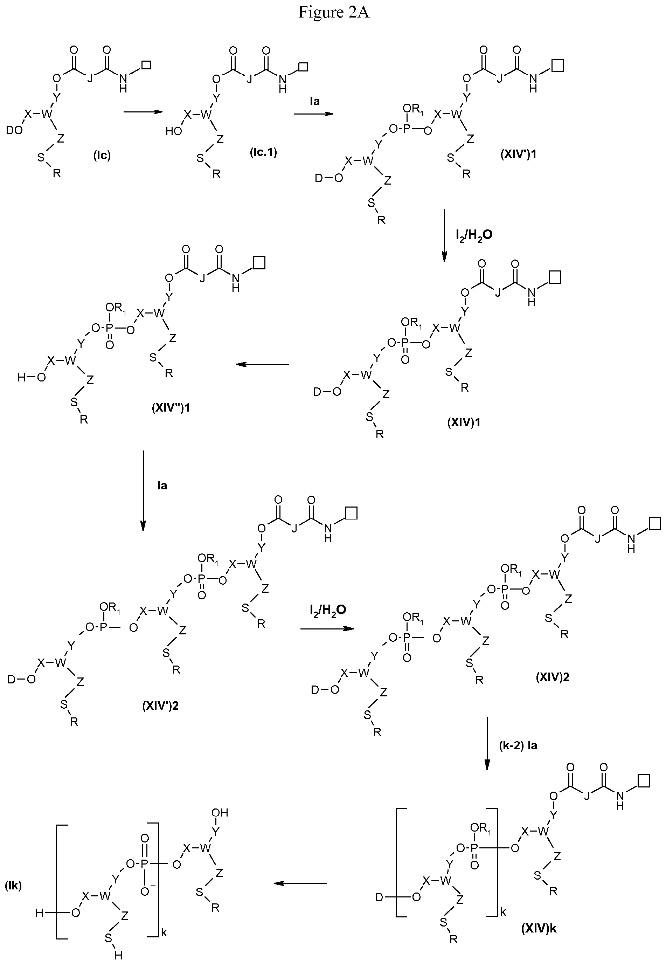

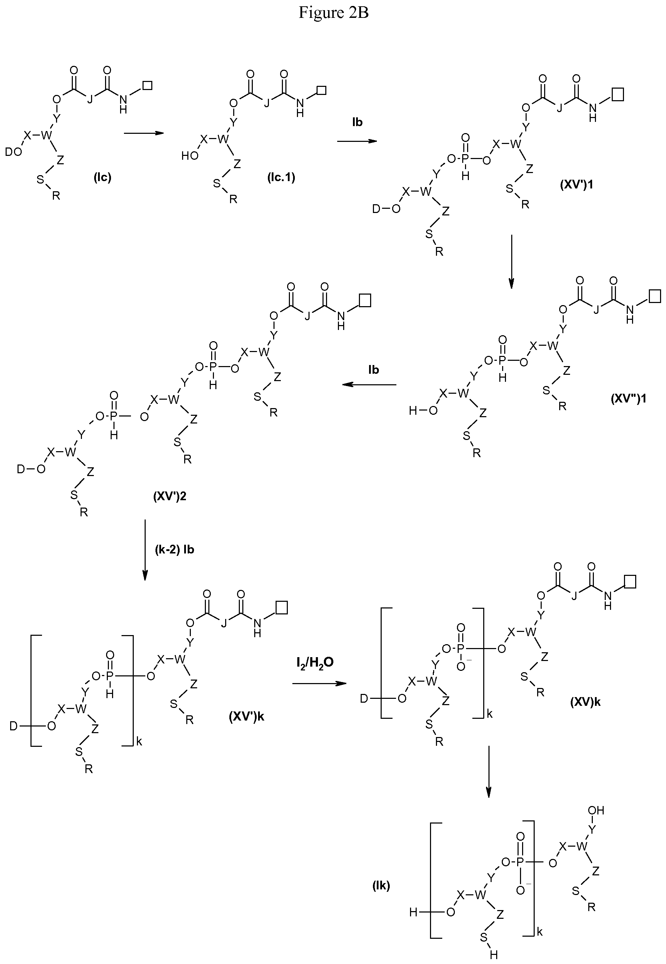

Un objet de la présente invention concerne un oligomère formé à partir de composés thiol de formule (I) précédemment décrits. Le procédé de synthèse de tels oligomères est décrit sur le schéma de la figure 2A pour l'oligomérisation de composés de formule (Ia) et sur le schéma de la figure 2B pour l'oligomérisation de composés de formule (Ib).

Dans une première étape, la fonction alcool du composé (Ic) est déprotégée pour conduire au composé (Ic.1). Cette étape de déprotection s'effectue par des moyens bien connus de l'homme du métier, de préférence en présence de l'acide di ou trichloroacétique pour les groupements DMTr et Pixyl et de pipéridine pour le groupement Fmoc.

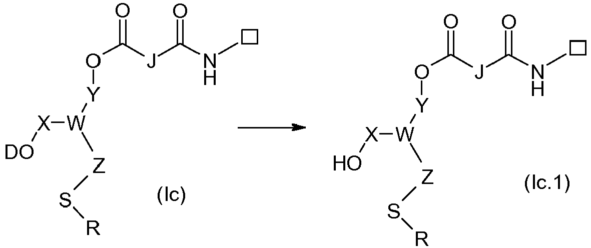

Ensuite, le composé (Ic.1) réagit avec le composé (Ia) ou (Ib) pour conduire respectivement au composé phosphitetriester (XIV')1 ou H-phosphonate diester (XV') 1

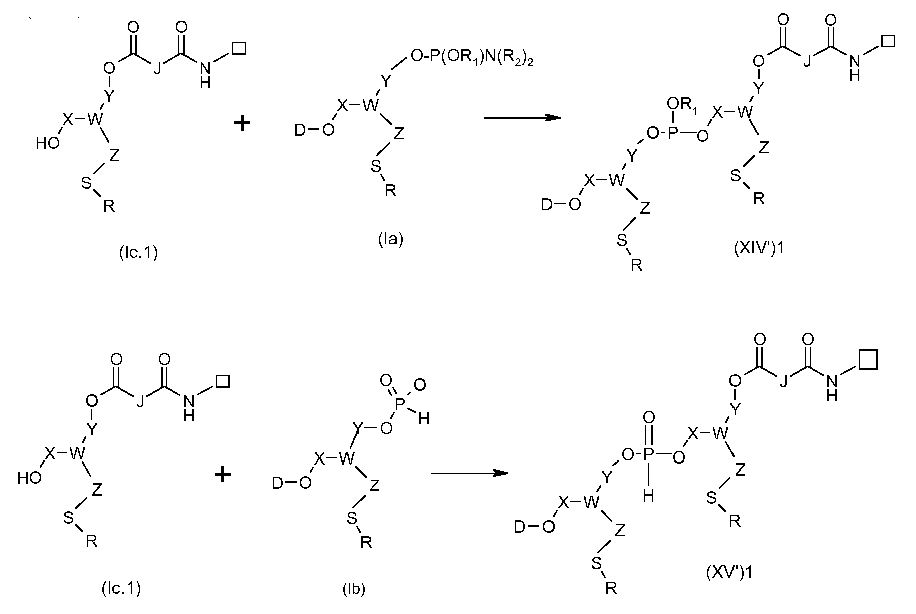

Puis, le composé (XIV')1 est oxydé de préférence en présence de diiode, le diode étant ensuite déplacé par de l'eau apportant l'atome d'oxygène du lien phosphate triester, pour conduire au composé phosphotriester (XIV)1. Cette étape d'oxydation s'effectue après chaque étape de couplage entre un composé (XIV)i et un composé (Ia).

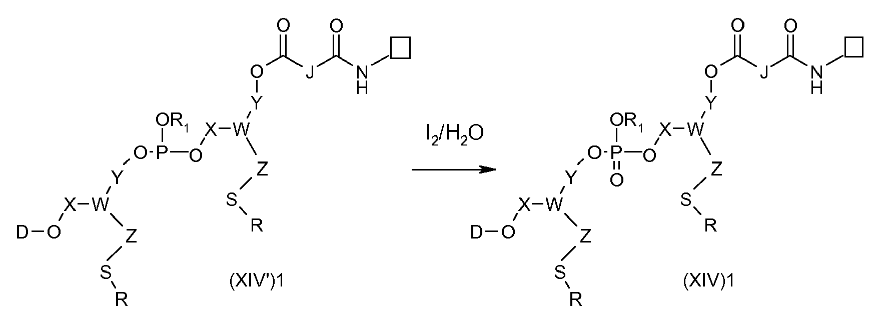

Puis, de la même manière, le composé (XIV)1 réagit avec (k-1) composés (Ia) pour conduire, après (k-1) oxydations, au composé (XIV)ₖ : 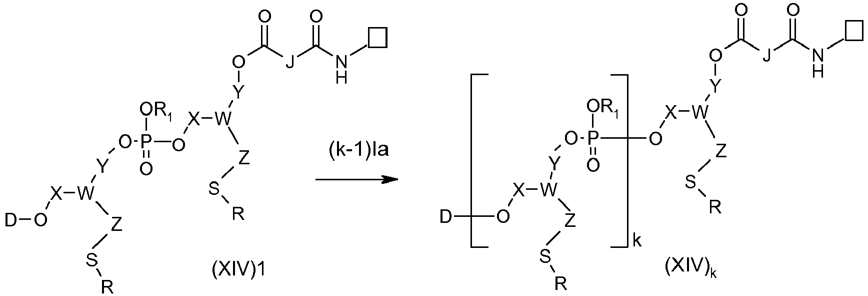 et le composé (XV')1 est déprotégé sur sa fonction alcool pour donner (XV")1 qui réagit avec (k-1) composés (Ib) pour conduire au composé (XV')ₖ :

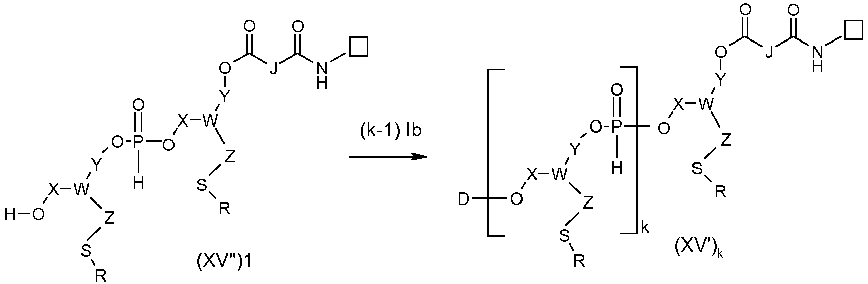

Puis, le composé (XV')ₖ est oxydé, de préférence en présence de diiode et d'eau pour conduire au composé phosphodiester (XV)ₖ.

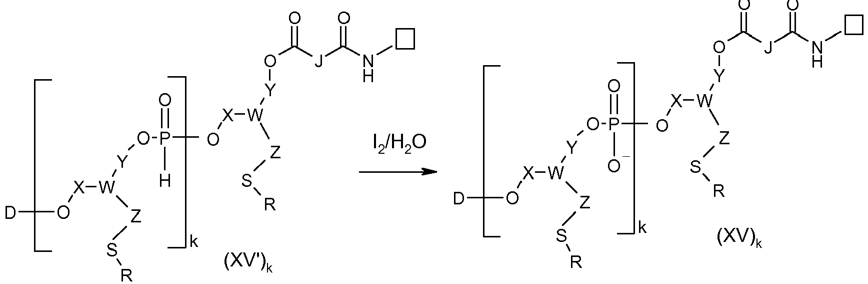

Enfin, une dernière étape éventuelle consiste à déprotéger les composés (XIV)ₖ ou (XV)ₖ pour conduire au même composé (I)ₖ.

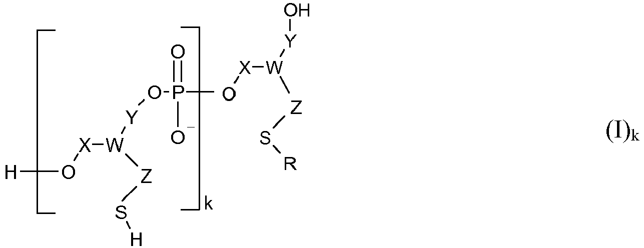

De préférence, l'oligomère résulte de l'oligomérisation de 2 à 12 composés (I), en particulier entre 2 et 8 composés (I), c'est-à-dire que l'oligomère peut comprendre 2, 3, 4, 5, 6, 7 ou 8 composés (I). De préférence, l'oligomère thiol destiné à être greffé sur une surface d'or comprend entre 3 et 8, avantageusement entre 4 et 8 composés (I) et l'oligomère thiol destiné à la conjugaison avec un substrat greffé par un groupement comportant au moins une double liaison carbone-carbone ou une triple liaison carbone-carbone ou un groupement halogénoacétamide, notamment du maléimide ou de l'acrylamide, comprend entre 2 et 6 composés (I).

Selon un mode de réalisation, l'oligomère est fabriqué uniquement à partir de composés de formule (Ia) ou de composés de formule (Ib). L'oligomérisation s'effectue par réaction entre la fonction alcool déprotégée d'un premier composé (I) et la fonction phosphoramidite ou H-phosphonate d'un second composé (I).

Il est également possible de prévoir la fabrication d'un oligomère à partir d'un mélange de composés (Ia) et de composés (Ib), ce mode de réalisation présentant moins d'intérêt.

De préférence, l'oligomère est fabriqué en utilisant la chimie des phosphoramidites, c'est-à-dire par oligomérisation de composés de type (Ia).

L'oligomérisation peut être effectuée sur support solide ou en solution. De préférence, l'oligomérisation est effectuée sur support solide. En effet, l'oligomérisation en solution implique des étapes de purifications par chromatographie, étapes non économiquement viables, en particulier pour les petites quantités requises dans les applications en diagnostic.

Un autre objet de l'invention décrite ci-dessus est un support solide greffé par un oligomère d'un composé (Ia) ou d'un composé (Ib) et répondant à la formule (XVI)ₖ ci-dessous :

(Ic) - (I')ₖ (XVI)ₖ

dans laquelle :
k représente un entier compris entre 1 et 11
(Ic) a la même signification que ci-dessus,
(I') représente (I'a) ou (I'b), avec : 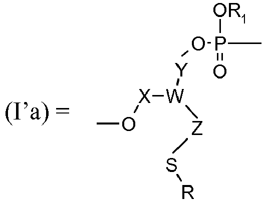 et

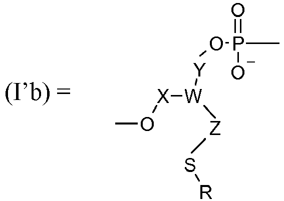

L'oligomère sur support solide □ formé à partir d'un composé (Ic) et de composés de formule (Ia) répond à la formule (XIV)ₖ suivante : 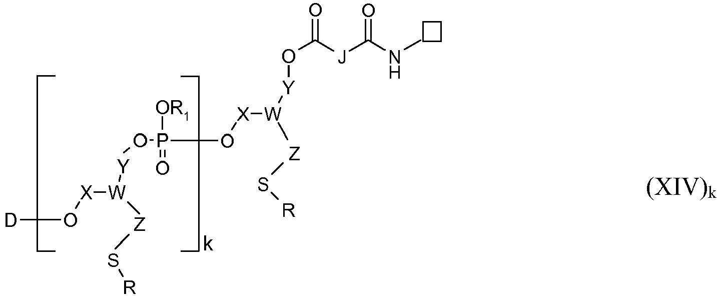 dans laquelle □, D, X, Y, W, Z, J, R et R₁ ont la même définition que ci-dessus et R peut en outre représenter H, k est un nombre entier compris entre 1 et 11.

L'oligomère sur support solide □ formé à partir d'un composé (Ic) et de composés de formule (Ib) répond à la formule (XV)ₖ suivante : 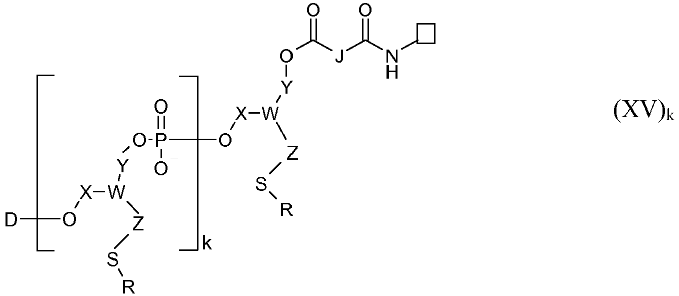 dans laquelle □, D, X, Y, W, Z, J et R ont la même définition que ci-dessus et R peut en outre représenter H, k est un nombre entier compris entre 1 et 11.

Dans le cas où l'oligomère est formé sur support solide □ à partir d'un composé (Ic) et de composés (Ib), le composé obtenu (XV)ₖ répond à la même formule que le composé (XIV)ₖ mais dans laquelle R₁ est un atome d'hydrogène après oxydation des liens H-phosphonate diesters.

A l'issue de la réaction d'oligomérisation, on peut prévoir de déprotéger les fonctions thiol par des méthodes classiques et alors R représente H.

### Oligonucléotides modifiés

Un autre objet de la présente invention concerne un oligonucléotide modifié, comprenant au moins un nucléotide, de préférence au moins deux nucléotides et au moins un composé thiol (I) précédemment décrit.

Dans la présente demande, un oligonucléotide désigne une chaîne comprenant entre 2 et 100 nucléotides.

Le greffage du composé thiol selon l'invention s'effectue en position 3', dans la chaîne, ou en position 5' d'un oligonucléotide.

Le procédé de préparation d'un tel oligonucléotide modifié comprend au moins :
- une étape de greffage d'un composé (I) sur un oligonucléotide pour donner un 5'-thiol oligonucléotide, ou
- une étape de greffage d'un nucléotide sur un oligomère d'un composé (I) pour donner un 3'-thiol oligonucléotide.

Selon un mode de réalisation de l'invention, l'oligonucléotide greffé répond à la formule (XIIa) suivante :

□- N₁ - N₂- ...- Nₙ₋₁- Nₙ- (I')_{y} -(M₁ -...- Mₘ₋₁- Mₘ)ₚ - (I')_{y'} (XIIa)

dans laquelle,
N₁, ...Nₙ représentent indépendamment les uns des autres un nucléotide,
M₁, ...Mₘ représentent indépendamment les uns des autres un nucléotide,
(I') représente un composé de formule (I'a) ou (I'b),
n est un nombre entier compris entre 1 et 100,
m est un nombre entier compris entre 1 et 100,
y est un nombre entier compris entre 1 et 12,
p représente 0 ou 1,
y' est un nombre entier compris entre 0 et 12 si p vaut 1 et, y' est égal à 0 si p vaut 0,
□ représente un support solide.

L'oligonucléotide modifié (XIIa) présente un ou plusieurs composés thiol en position 5' d'un oligonucléotide N ou bien dans la chaîne nucléotidique. Ce composé est obtenu par un greffage d'un composé (I) suivi par une élongation de l'oligomère issu de (I) en position 5' d'un oligonucléotide. Puis, dans le cas où p = 1, l'élongation de la chaîne nucléotidique se poursuit. Puis, de la même manière, on peut envisager le greffage d'un ou plusieurs composés (I) supplémentaires en position 5' de l'oligonucléotide M.

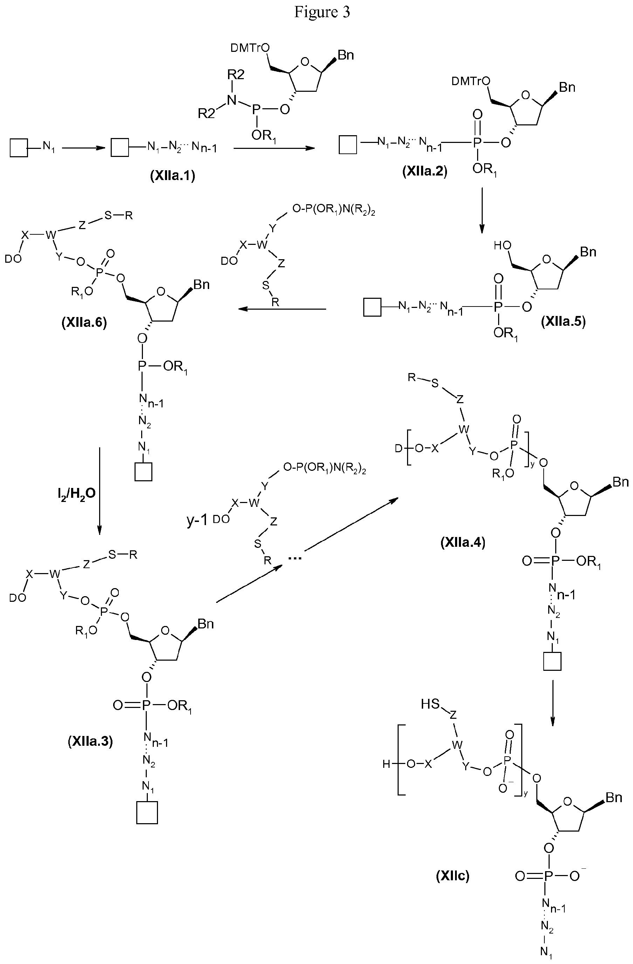

Un schéma d'obtention du composé (XIIa) est décrit sur la figure 3 sur laquelle les composés thiol sont de type (Ia) et p est égal à 0. Les trois premières étapes de fabrication du composé (XIIa) permettent la synthèse d'un oligonucléotide. La synthèse de l'oligonucléotide est effectuée sur support solide par une méthode classique bien connue de l'homme du métier. Lors de la première étape, un premier nucléotide est greffé sur un support solide, puis les autres nucléotides sont greffés suivant des méthodes de synthèse bien connues de l'homme du métier. Le composé suivant est obtenu :

□-N₁-N₂···Nₙ₋₁ (XIIa.1)

Puis, un autre nucléotide est greffé par une méthode identique pour conduire au composé de formule (XIIa.2).

Lors de l'étape suivante, un composé thiol selon l'invention de type (Ia) est greffé en position 5' de l'oligonucléotide (XIIa.2) pour conduire au composé (XIIa.3) :

□ - _{N1} - N₂- _{...}- Nₙ₋₁- Nₙ (I'a) (XIIa.3)

Lors de cette étape, le greffage se fait de manière classique par réaction de la fonction phosphoramidite du composé (Ia) avec la fonction alcool en position 5' du nucléotide terminal du composé (XIIa.2).

Sur le schéma de la figure 3, un exemple de synthèse est décrit avec l'oligomérisation du composé thiol de type (Ia) ; un procédé de synthèse similaire est utilisé pour la synthèse d'oligonucléotides modifiés avec des composés thiol de type (Ib).

Ensuite, l'oligomérisation telle que décrite précédemment, notamment sur les figures 2A et 2B, se poursuit à partir du composé (XIIa.3) ci-dessus, par réaction avec un ou plusieurs composés (Ia) ou (Ib) pour conduire au composé (XIIa.4) de formule :

□ - N₁ - N₂- ...- Nₙ₋₁- Nₙ (I'a)_{y}

ou en formule développée (dans le cas où p = 0) : 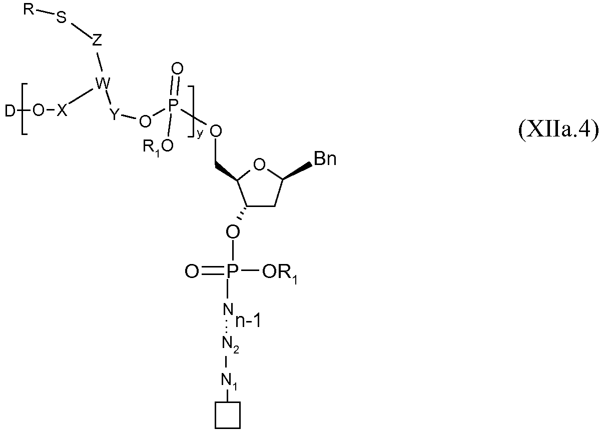 dans laquelle,
□, D, R, X, Y, W, Z, R₁ ont la même définition que pour le composé (I) et R₁ peut en outre représenter H,
n, y et N₁, N₂, ...Nₙ₋₁ ont la même définition que pour le composé (XIIa),
Bn représente une base classiquement utilisée dans une chaîne nucléotidique.

Dans le cas où l'élongation de l'oligomère s'effectue avec des composés de type (Ib), l'oligonucléotide modifié présente une structure similaire à celle de l'oligonucléotide modifié (XIIa.4) mais dans laquelle R₁ représente H.

Un greffage ultérieur de composés nucléotides M₁, M₂, ...Mₘ est ensuite possible pour aboutir au produit (XIIa) avec p = 1. Dans ces cas, l'élongation s'effectue également sur support solide.

Cette étape de greffage s'effectue de manière classique par des méthodes connues de l'homme du métier.

Selon un autre mode de réalisation de l'invention, l'oligonucléotide greffé répond à la formule (XIIIa) suivante :

(Ic) - (I')_{y-1} - N₁ - N₂- ...- Nₙ₋₁- Nₙ - (I')_{y'} -[M₁ - M₂ - ... - Mₘ₋₁ - Mₘ]ₚ-(I')_{y"} (XIIIa)

dans laquelle,
N₁, ...Nₙ représentent indépendamment les uns des autres un nucléotide,
M₁, ... Mₘ représentent indépendamment les uns des autres un nucléotide,
(I') représente un composé de formule (I'a) ou (I'b),
n est un nombre entier compris entre 1 et 100,
m est un nombre entier compris entre 1 et 100,
y est un nombre entier compris entre 1 et 12,
y' est un nombre entier compris entre 0 et 12,
p vaut 0 ou 1 si y' est différent de 0 et, si y' vaut 0 alors p vaut 0,
y" est un nombre entier compris entre 0 et 12 si p vaut 1 et, si p vaut 0 alors y" vaut 0.

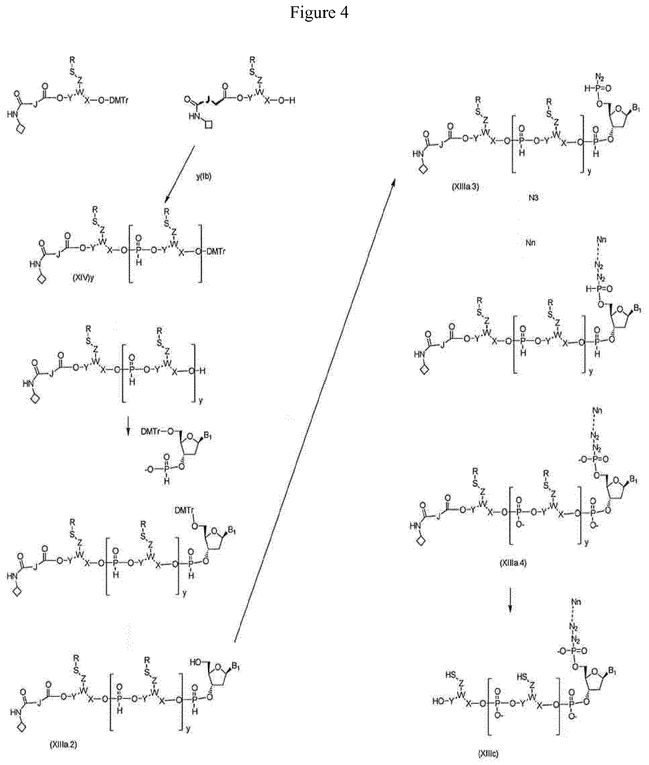

Un schéma représentant un mode de synthèse du composé (XIIIc) est décrit sur la figure 4 en utilisant un oligomère formé à partir d'un composé de type (Ic) où J est un groupement éthyle, et de composés de type (Ib).

La synthèse du composé de formule (XIIIc) comprend une première étape consistant en l'oligomérisation du composé thiol selon l'invention de formule (I) dont le procédé est décrit précédemment pour conduire au composé de formule (XIIIa.1):

(Ic) - (I')_{y-1} (XIIIa.1)

dans laquelle,
(Ic) a la même définition que précédemment,
(I') représente un groupement de type (I'a) ou (I"b), où 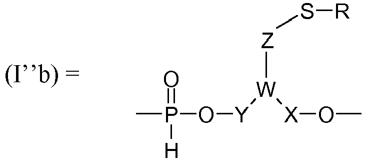 ou en formule développée dans le cas où (I') représente (I"b) :

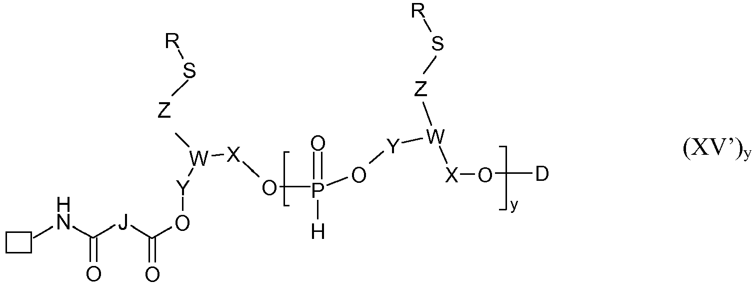

Dans une seconde étape, un premier nucléotide N₁ est greffé sur l'oligomère de formule (XIIIa.1) pour conduire au composé de formule (XIIIa.2) ci-dessous :

(Ic) - (I')_{y-1}- N₁ (XIIIa.2)

Cette étape de greffage s'effectue par réaction entre la fonction alcool déprotégée au bout de la chaîne d'oligomère du composé (XIIIa.1) et la fonction phosphoramidite ou H-phosphonate du premier nucléotide N₁.

L'oligonucléotide modifié est ensuite synthétisé par n'importe quelle méthode connue de l'homme du métier, notamment une méthode classique bien connue de l'homme du métier par réaction entre la fonction alcool en 5' du premier nucléotide présent sur l'oligomère du composé thiol et la fonction phosphoramidite ou H-phosphonate en position 3' d'un second nucléotide. La synthèse se poursuit par des étapes successives similaires d'élongation de la chaîne nucléotidique bien connues de l'homme du métier pour conduire au composé de formule (XIIIa).

Un autre objet de la présente invention concerne les composés obtenus à partir des composés de formule (XIIa) et (XIIIa) ci-dessus après coupure de la liaison qui relie l'oligonucléotide modifié au support solide. La coupure de la liaison s'effectue au niveau de la fonction ester.

Ainsi, selon un mode de réalisation de l'invention, l'oligonucléotide non supporté présente la structure (XIIb) suivante :

N₁ - N₂- ...- Nₙ₋₁- Nₙ- (I')_{y} -(M₁ -...- Mₘ₋₁- Mₘ)ₚ - (I')_{y'} (XIIb)

dans laquelle,
N₁, ...Nₙ représentent indépendamment les uns des autres un nucléotide,
M₁, ... Mₘ représentent indépendamment les uns des autres un nucléotide,
(I') représente un composé de formule (I'a) ou (I'b),
n est un nombre entier compris entre 1 et 100,
m est un nombre entier compris entre 1 et 100,
y est un nombre entier compris entre 1 et 12,
p représente 0 ou 1,
y' est un nombre entier compris entre 0 et 12 si p vaut 1 et, y' est égal à 0 si p vaut 0.

Le retrait du support s'effectue en deux étapes avec premièrement une base forte non nucléophile (piperidine ou DBU) pour éliminer les groupements cyanoéthyles si présents (cas des phosphoramidites) et deuxièmement par un procédé classique connu de l'homme du métier, de préférence par un traitement du composé (XIIa) avec de l'hydroxyde d'ammonium (NH₄OH). Il est nécessaire d'éliminer les groupements cyanoéthyles avant la déprotection des groupements thiols car ils forment de l'acrylonitrile lors de leur élimination qui réagit fortement avec les fonctions thiol.

Selon un autre mode de réalisation de l'invention, l'oligonucléotide non supporté présente la structure (XIIIb) suivante :

(Ic')-(I')_{y-1}- N₁ - N₂- ...- Nₙ₋₁- Nₙ - (I')_{y'} -[M₁ - M₂ - ... - Mₘ₋₁- Mₘ]ₚ-(I')_{y"} (XIIIb)

dans laquelle,
N₁, ...Nₙ représentent indépendamment les uns des autres un nucléotide,
M₁, ... Mₘ représentent indépendamment les uns des autres un nucléotide,
(Ic') représente le composé obtenu à partir de (Ic) par coupure de la liaison ester avec le support solide □,
(I') représente un composé de formule (I'a) ou (I'b),
n est un nombre entier compris entre 1 et 100,
m est un nombre entier compris entre 1 et 100,
y est un nombre entier compris entre 1 et 12,
y' est un nombre entier compris entre 0 et 12,
p vaut 0 ou 1 si y' est différent de 0 et, si y' vaut 0 alors p vaut 0,
y" est un nombre entier compris entre 0 et 12 si p vaut 1 et, si p vaut 0 alors y" vaut 0.

Le retrait du support s'effectue par un procédé classique connu de l'homme du métier, de préférence par un traitement du composé (XIIIa) avec de l'hydroxyde d'ammonium (NH₄OH).

Un autre objet de la présente invention concerne les oligonucléotides modifiés (XIIc) et (XIIIc) obtenus respectivement à partir des composés (XIIa) et (XIIIa) par déprotection de la fonction thiol et coupure de la liaison reliant le composé au support solide (Figures 3 et 4 respectivement).

Dans le cas où l'oligonucléotide est modifié par un ou plusieurs composés thiol de type (Ia), après déprotection de la fonction thiol et de la fonction phosphoramidite du composé (XIIa) par un traitement connu de l'homme du métier, le composé de formule (XIIc) est obtenu :

N₁ - N₂- ...- Nₙ₋₁- Nₙ- (I")_{y} -(M₁ -...- Mₘ₋₁- Mₘ)ₚ - (I")_{y'} (XIIc)

dans laquelle,
N₁, ...Nₙ représentent indépendamment les uns des autres un nucléotide,
M₁, ...Mₘ représentent indépendamment les uns des autres un nucléotide, 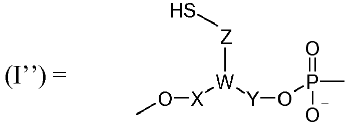
n est un nombre entier compris entre 1 et 100,
m est un nombre entier compris entre 1 et 100,
y est un nombre entier compris entre 1 et 12,
p représente 0 ou 1,
y' est un nombre entier compris entre 0 et 12 si p vaut 1 et, y' est égal à 0 si p vaut 0.
ou en formule développée dans le cas où p=0 : 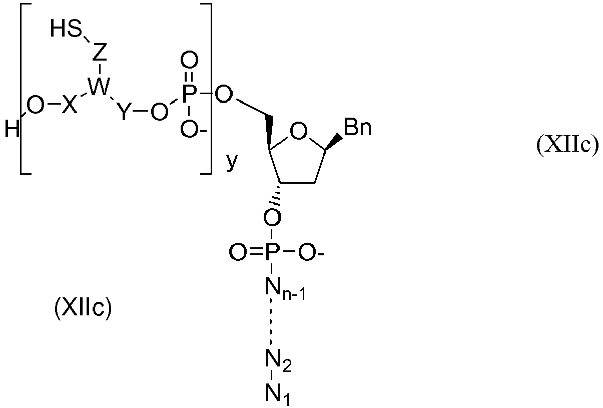 dans laquelle,
X, Y, W, Z ont la même définition que pour le composé (I),
N₁, ...Nₙ, n, y ont la même définition que pour le composé (XIIa),
Bn représente une base classiquement utilisée dans une chaîne nucléotidique.

Dans le cas où l'oligonucléotide est modifié par un ou plusieurs composés thiol de type (Ib), après déprotection de la fonction thiol du composé (XIIIa) par un traitement connu de l'homme du métier, le composé de formule (XIIIc) est obtenu :

(Ic')-(I')_{y-1}- N₁ - N₂- ...- Nₙ₋₁- Nₙ - (I")_{y}, -[M₁ - M₂ - -... -Mₘ₋₁-Mₘ]ₚ-(I")_{y"} (XIIIc)

dans laquelle,
N₁, ...Nₙ représentent indépendamment les uns des autres un nucléotide,
M₁, ... Mₘ représentent indépendamment les uns des autres un nucléotide,
(Ic') représente le composé obtenu à partir de (Ic) par coupure de la liaison ester avec le support solide □, 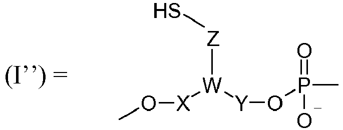
n est un nombre entier compris entre 1 et 100,
m est un nombre entier compris entre 1 et 100,
y est un nombre entier compris entre 1 et 12,
y' est un nombre entier compris entre 0 et 12,
p vaut 0 ou 1 si y' est différent de 0 et, si y' vaut 0 alors p vaut 0,
y" est un nombre entier compris entre 0 et 12 si p vaut 1 et, si p vaut 0 alors y" vaut 0.
ou en formule développée dans le cas où y' = p = 0 : 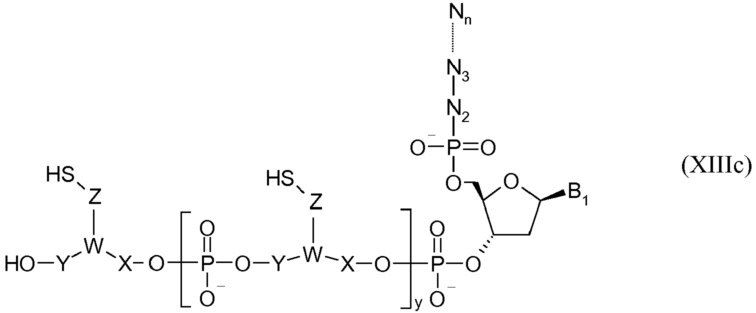 dans laquelle,
X, Y, W, Z ont la même définition que pour le composé (I),
N₂, ...Nₙ, n et y ont la même définition que pour le composé (XIIa),
Bn correspond à une base classiquement utilisée dans une chaîne nucléotidique.

Lors de la synthèse de l'oligonucléotide, la fonction thiol est de préférence protégée. En effet, la fonction thiol peut réagir avec la fonction phosphoramidite entrante.

Selon un mode de réalisation, l'étape de déprotection des fonctions thiol et d'élimination du support solide s'effectue en une seule étape de traitement de l'oligonucléotide modifié (XIIa) ou (XIIIa).

Selon un mode de réalisation, l'élimination du support solide s'effectue dans une première étape puis la déprotection des fonctions thiol s'effectue dans une seconde étape.

L'oligonucléotide final (XIIc) (respectivement l'oligonucléotide final (XIIIc)) est obtenu indépendamment du composé thiol de départ, que ce soit à partir du composé (Ia) ou du composé (Ib). En effet, quel que soit le monomère (Ia) ou (Ib) utilisé, l'unité monomère thiol résultant de la réaction d'oligomérisation correspond au composé (I") décrit précédemment.

Les supports greffés de formules (XVI)ₖ selon l'invention permettent d'amorcer la synthèse d'oligonucléotides. On peut notamment prévoir de préparer de façon industrielle ou semi-industrielle des supports solides greffés par des séquences d'oligomères qui seront employées comme séquence polythiol en position 3' d'un oligonucléotide.

### Kit de synthèse

Un tel support solide greffé par un premier oligomère (Ic) - (I')ₖ peut avantageusement être utilisé dans un kit de synthèse d'oligonucléotides modifiés. Dans un tel kit, il peut être associé avec un support comportant des zones de réception telles que les puits d'une plaque multi-puits dans lesquels des réactions de synthèse sont faites par des méthodes telles que celles qui sont connues pour la réalisation des réactions d'oligomérisation de nucléotides.

### Dispositif automatisé

Un tel kit peut être utilisé dans un dispositif automatisé pour la synthèse d'oligonucléotides. Dans ce cas, un tel dispositif comporte au moins
- des réservoirs distincts contenant :
   - des nucléotides,
   - des activateurs de couplage et
   - des produits de lavage,
- des moyens mécaniques de prélèvement et de distribution d'échantillons de produits, ainsi que des moyens informatiques permettant la mise en oeuvre contrôlée de ces moyens mécaniques, ainsi que :
- au moins un réservoir dans lequel est placé un support solide greffé par un oligomère (XVI)ₖ des composés (I) tels que décrit ci-dessus, et/ou au moins un réservoir contenant un composé (Ia) ou un composé (Ib).

Un tel automate comporte également des moyens mécaniques de prélèvement d'échantillons dans les différents réservoirs et des moyens de distribution de ces échantillons dans le réservoir comportant le support solide. De tels moyens peuvent consister en un ensemble de tuyaux de circulation de fluides et valves, arrangés éventuellement en circuits microfluidiques, ou en des bras articulés équipés de pipettes de prélèvement. Le dispositif automatisé comporte aussi des moyens informatiques (logiciels) permettant la mise en oeuvre contrôlée de ces moyens mécaniques, permettant la mise en oeuvre de réactions séquencées.

### Fonctionnalisation de surface

Les oligonucléotides modifiés selon l'invention peuvent être déposés sur un substrat afin de fonctionnaliser la surface de ce substrat. Le substrat peut être métallique ou bien en polymère.

Selon un mode de réalisation, le substrat est métallique, par exemple en cuivre ou en titane, et est recouvert partiellement ou sur toute sa surface d'un film d'or ou de platine, de préférence d'or.

Selon un autre mode de réalisation, le substrat en polymère, par exemple en polystyrène, est greffé par des fonctions alcényles ou alcynyles ou bromoacétamides ou iodoacétamides.

Selon un mode de réalisation, le substrat en polymère est un polymère conducteur.

Selon un mode de réalisation, les fonctions alcényles ou alcynyles sont activées par une fonction carbonyle en alpha, de préférence les fonctions alcényles ou alcynyles sont choisies parmi les groupements maléimide, acrylamide, iodoacétamido ou bromoacétamido, 2-propynamide ou N-alkyl-2-propynamide.

Par exemple le substrat peut comporter des zones de réception recouvertes d'un film d'or ou de platine ou recouvertes de fonctions alcényles ou alcynyles, telles que des fonctions maléimide ou acrylamide sur lesquelles l'oligonucléotide modifié est déposé.

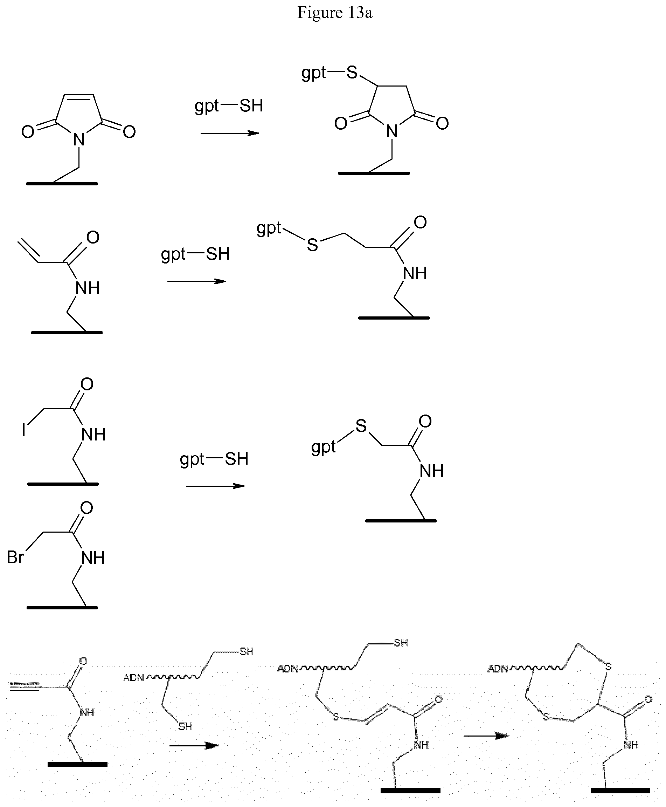

Comme illustré sur la figure 13a, dans laquelle le composé gpt - SH représente l'oligonucléotide modifié (XIIc) ou l'oligonucléotide modifié (XIIIc) selon l'invention, la fonction thiol réagit avec la double liaison carbone-carbone ou la triple liaison carbone-carbone activée par une fonction carbonyle en alpha. Du haut vers le bas de la figure 13a, la première réaction de fonctionnalisation correspond à une surface greffée maléimide, la seconde réaction correspond à une surface greffée acrylamide, la troisième réaction correspond à une surface greffée iodoacétamido ou bromoacétamido et la quatrième réaction correspond à une surface greffée 2-propynamide effectuée dans le cas d'un oligonucléotide (XIIc) ou (XIIIc) modifié par deux composés thiols selon l'invention.

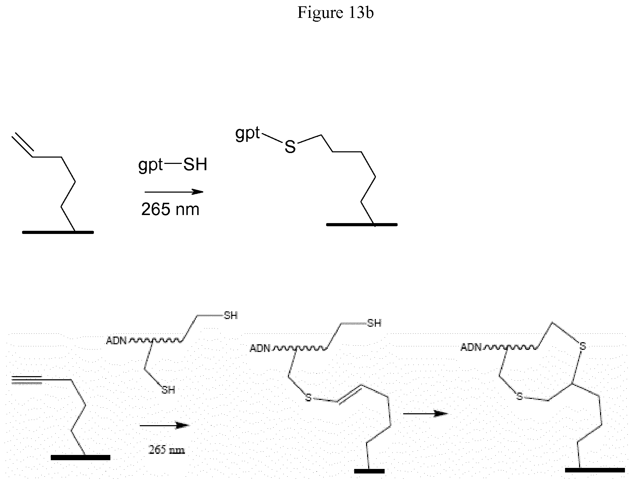

Dans le cas de la figure 13b, la surface est greffée par des groupements alcényles (1^{ère} réaction) et alcynyles (2^{nde} réaction) non activés. La réaction entre la fonction thiol de l'oligonucléotide modifié (XIIc) ou (XIIIc) s'effectue à l'aide d'une activation lumineuse (λ = 265 nm). La première réaction de fonctionnalisation est effectuée à l'aide d'un oligonucléotide modifié monothiol schématiquement représenté par gpt - SH et la seconde réaction est effectuée à l'aide d'un oligonucléotide modifié dithiol.

Dans le cas ou le support est greffé par des fonctions alcynyles, la fonctionnalisation de surface par un oligonucléotide modifié polythiol est très intéressante car elle conduit à une structure cyclique grâce à deux réactions successives entre une première fonction thiol et la fonction -yne dans une première étape puis entre une deuxième fonction thiol et la fonction -ène résultante dans une seconde étape (figures 13a et 13b).

Selon un mode de réalisation préféré, le substrat est greffé par des groupements maléimide ou acrylamide.

Ainsi, l'invention permet par exemple de fonctionnaliser une surface d'or par création de liaison(s) or-soufre entre la surface du substrat et l'oligonucléotide modifié ou bien de fonctionnaliser une surface greffée par des fonctions maléimide ou acrylamide par création de liaison(s) thioéther.

La fixation des oligonucléotides modifiés à la surface du substrat s'effectue par contact de la surface à traiter avec une solution comprenant l'oligonucléotide modifié, tel que ceux représentés par les formules (XIIc) et (XIIIc). On prévoit généralement ultérieurement une ou plusieurs étapes de lavage et de séchage. En général, la solution d'oligonucléotides modifiés est à une concentration comprise entre 0,10µM et 500 µM, de préférence entre 0,50 µM et 100 µM pour la surface d'or et entre 50 et 200 nM, de préférence entre 75 nM et 150 nM pour le maléimide ou l'acrylamide suivi d'un lavage pour éliminer ce qui n'a pas réagi.

La présence de plusieurs atomes de soufre sur l'oligonucléotide permet de créer plusieurs liaisons or-soufre, ou plusieurs liaisons thioéthers, ce qui permet de stabiliser l'oligonucléotide sur la surface.

Selon un mode de réalisation, le substrat est non planaire, par exemple sous forme de microparticules ou nanoparticules. Les oligonucléotides modifiés selon l'invention peuvent alors être greffés sur ces microparticules ou nanoparticules. De préférence, ces particules sont magnétiques. En effet, ces particules magnétiques peuvent alors être facilement amenées à la surface d'une électrode ou au fond des puits d'une microplaque, en vue d'un test de détection, par application d'un aimant.

### Fixation à des marqueurs ou ligands

Les oligomères de la présente invention peuvent également être utilisés afin de lier par un ou plusieurs points d'attachement des marqueurs ou des ligands à des molécules ou des polymères. Les marqueurs ou ligands peuvent être par exemples, des marqueurs enzymatiques, chromogènes, fluorogènes, radioactifs, ou chemiluminescents, des métaux, des ions métalliques, des hormones, des protéines, des peptides, des saccharides, oligosaccharides, des agents nucléolytiques ou protéolytiques, des agents de liaison, tels que une biotine, un antigène, un haptène, un anticorps ou un récepteur. Les marqueurs ou ligands peuvent être tous des composés d'intérêt biologique qui peuvent influencer le transport d'oligonucléotides à travers des membranes biologiques et/ou qui peuvent modifier la solubilité des oligonucléotides.

### Kits de test

Un autre objet de l'invention consiste en un kit de test et/ou de diagnostic comportant au moins un support comportant au moins une zone de réception, sur laquelle est placé au moins un oligonucléotide modifié selon l'invention.

Ainsi, le kit de test peut être utilisé pour le criblage de molécules biologiquement actives ou pour des tests de diagnostic ou séquençage. On peut prévoir que le kit de diagnostic comporte plusieurs zones de réception distinctes, sur lesquelles un support solide greffé par des oligonucléotides identiques ou distincts est déposé. Par exemple, des supports solides greffés par des oligonucléotides identiques ou distincts peuvent être placés sur des zones de réception distinctes d'un substrat recouvert d'un film d'or ou d'un substrat greffé par des fonctions comportant au moins une double liaison carbone-carbone ou une triple liaison carbone-carbone ou des fonctions halogénoacétamide, de préférence des fonctions maléimide ou acrylamide de façon à former un support de test et/ou de diagnostic.

Le support peut notamment être une électrode d'or. Le kit de test peut comprendre une cellule électrochimique comportant une électrode de travail, une contre électrode et une électrode de référence. L'électrode de travail peut être en or et la contre électrode en platine et l'électrode de référence en argent. L'étude de l'interaction d'un oligonucléotide modifié avec une molécule à tester peut comporter une étape de voltampérométrie cyclique.

Le greffage avec des fonctions alcényle, alcynyle ou halogénoacétamide, telles que maléimide ou acrylamide, peut par exemple être utilisé pour des applications dans le domaine du diagnostic en format microplaque et/ou pour la mise en oeuvre de tests de type ELOSA (« Enzyme-Linked OligoSorbent Assay » en anglais ou « dosage d'oligoadsorption par enzyme liée » en français). Au cours de ce type de test, la surface des puits est greffée par des fonctions alcényle, alcynyle ou halogénoacétamide, telle que des fonctions maléimide ou acrylamide. Puis, la surface est mise en contact avec des oligonucléotides modifiés selon l'invention. Ainsi, une ou plusieurs liaisons thioéther se forment grâce à la présence d'un ou plusieurs composés thiol selon l'invention sur les oligonucléotides. Puis, le test consiste à mettre en contact un échantillon à tester avec les puits ainsi fonctionnalisés, pour notamment mesurer l'hybridation des oligonucléotides. La mesure peut par exemple se faire par fluorescence grâce à un marquage des chaînes d'oligonucléotides.

### EXEMPLES

Dans les exemples, on entend par « sonde », une chaîne d'oligonucléotides comprenant au moins un composé thiol selon l'invention destinée à être immobilisée sur une surface.

On entend par « cible », une chaîne d'oligonucléotides destinée à s'hybrider avec la sonde, par exemple, lors d'un test de diagnostic.

### Synthèse du composé 1-O-(4,4'-diméthoxytrityl)-2-(6-S-acétylthiohexyloxyméthyl)-2-méthylpropane-1,3-diol 4

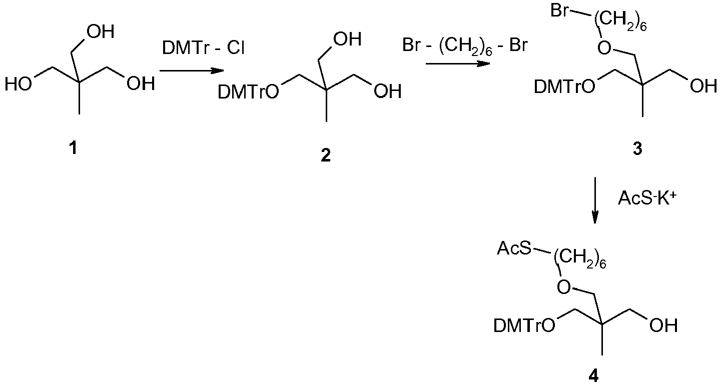

Le composé **3** est obtenu à partir du composé **1** en suivant le protocole décrit dans Pourceau, G., Meyer, A., Vasseur, J. J., and Morvan, F., Journal of Organic Chemistry 74, 2009, 1218-1222.

A une solution de 1-*O*-(4,4'-diméthoxytrityl)-2-(6-bromohexyloxyméthyl)-2-méthyl-1,3-propanediol **3**, (556 mg, 0,95 mmol) et de thioacétate de potassium (162 mg, 1,42 mmol) dans du toluène anhydre (10 mL) on ajoute de l'éther couronne 18-6 (70 mg, 0,26 mmol). Le mélange est agité magnétiquement 2 heures à 50 °C. Après dilution avec du dichlorométhane (150 mL) le mélange est filtré et la phase organique lavée avec de l'eau (2 × 50 mL) puis séchée sur Na₂SO₄. Après évaporation, le brut de réaction est purifié par chromatographie sur silice (0 à 30% d'acétate d'éthyle dans le cyclohexane) pour conduire au produit désiré sous forme d'une huile incolore (413 mg, 75%).

### Synthèse du 1-O-(4,4'-diméthoxytrityl)-2-(6-S-acétylthiohexyloxyméthyl)-2-méthyl-3-O-(2-cyanoéthyl-N,N'-diisopropylphosphoramidite)-propane-1,3-diol 5

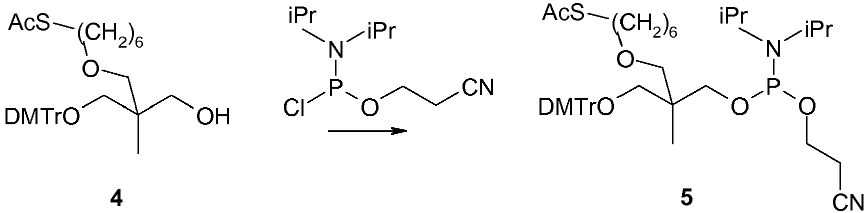

A une solution de 1-*O*-(4,4'-diméthoxytrityl)-2-(6-S-acétylthiohexyloxyméthyl)-2-méthylpropane-1,3-diol **4** (413 mg, 0,71 mmol) et de diisopropyléthylamine (186 µL, 1,06 mmol) dans le dichlorométhane anhydre (10 mL) est ajouté du 2-cyanoéthyl-N,N'-diisopropylchlorophosphoramidite (190 µL, 0,85 mmol). Le mélange est agité magnétiquement une heure à température ambiante. L'excès de réactif est neutralisé par addition de 500 µL d'eau puis le mélange est dilué avec du dichlorométhane (150 mL). La phase organique est lavée avec une solution aqueuse saturée en NaHCO₃ (100 mL), puis séchée sur Na₂SO₄. Après évaporation, le brut est purifié par chromatographie sur silice (0 à 30% d'acétate d'éthyle dans du cyclohexane contenant 4% de triéthylamine) conduisant au composé **5** désiré sous forme d'une huile incolore (400 mg, 72%).

### Synthèse du support solide thiol 6

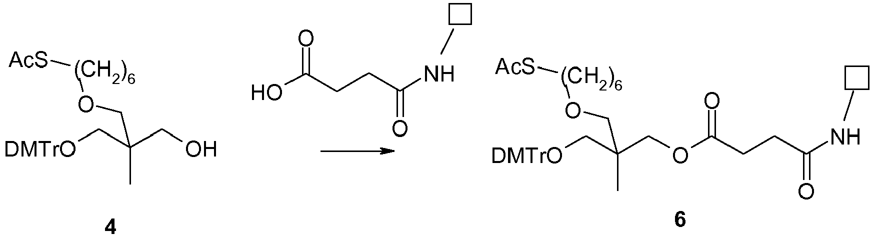

Dans un tube rodé, le 1-O-(4,4'-diméthoxytrityl)-2-(6-S-acétylthiohexyloxyméthyl)-2-méthyl-1,3-propanediol **4** (178 mg, 0,3 mmol) et la dimethylaminopyridine (DMAP 36 mg, 0,3 mmol) sont coévaporés avec 3 mL de pyridine anhydre. Puis on ajoute la succinyl-longue chaine alkylamine -CPG (Controled Pore Glass) (1 g), la pyridine anhydre (5 mL), la triéthylamine anhydre (160mL, 1,2 mmol) et l'éthyldiméthylaminopropyl carbodiimide (EDC, 280 mg, 2,0 mmol). Puis on rince avec 1 mL de pyridine anhydre. Le mélange est agité une nuit.

Le mélange est filtré et lavé avec CH₂Cl₂ (10 mL) puis séché au dessiccateur. Le composé thiol sur support solide est traité par une solution d'anhydride acétique, N-méthylimidazole, 2,6-lutidine dans le THF pendant 3h sous agitation. Le mélange est filtré et lavé avec CH₂Cl₂ (10 mL) puis séché au dessiccateur pour donner le support solide thiol **6** (940 mg) avec une fonctionnalisation de 29 µmol/g.

### Préparation d'oligonucléotides modifiés

Trois oligonucléotides comprenant un groupement ferrocène en position 5' et un nombre croissant de composés thiol de type (Ia) selon l'invention (1, 2 et 4 composés thiol) ont été synthétisés sur un synthétiseur d'ADN. Le groupement ferrocène a été utilisé afin de visualiser l'immobilisation de l'oligonucléotide sur la surface d'or par voltampérométrie cyclique. Un, deux ou quatre groupements thiols ont été introduits sur un support solide de type propanediol et la séquence ADN SEQ ID NO : 3 a été greffée. Enfin, une phosphoramidite alpha-thymidine portant un groupement ferrocène a été introduite en position 5' de l'oligonucléotide modifié. La déprotection qui suit s'effectue en deux étapes. D'abord, le milieu est traité avec 10% de pipéridine dans l'acétonitrile pendant 10 minutes afin d'éliminer le groupement cyanoéthyle par une bêta-élimination et l'acrylonitrile résultant est éliminé du milieu par un lavage à l'acétonitrile. Puis, un traitement à l'hydroxyde d'ammonium concentré permet d'éliminer les groupements protecteurs acyles sur les nucléobases et sur les fonctions thiol et permet d'hydrolyser le bras de liaison succinyle (support solide). Ce protocole permet d'éviter l'addition de Michael entre les fonctions thiol déprotégées et l'acrilonitrile. Après évaporation, l'oligonucléotide modifié non supporté est purifié par chromatographie HPLC en phase inverse sur colonne C18.

### Tétrathiol (oligonucléotide modifié par 4 composés thiol)

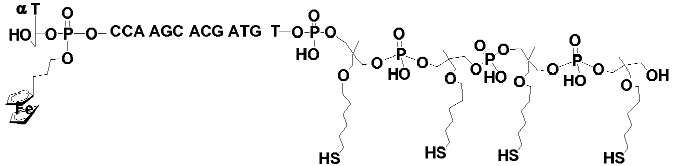

### Dithiol (oligonucléotide modifié par 2 composés thiol)

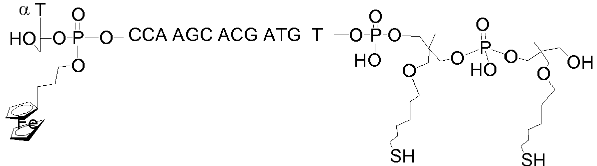

### Monothiol (oligonucléotide modifié par 1 composé thiol)

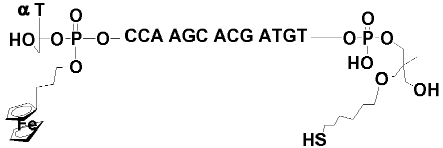

### Greffage et étude de stabilité des oligonucléotides modifiés (tétrathiol, dithiol et monothiol)

Pour cette étude, un potentiostat multivoies VMP3 Biologic (Biologic Science Instruments, Pont de Claix) a été utilisé. Les résultats ont été enregistrés à l'aide du logiciel EC-Lab de Biologic Science Instruments.

La cellule électrochimique est composée d'une électrode d'or de 0,28cm² de surface, d'une contre électrode en platine et d'une électrode de référence Ag/AgCl.

### Etape 1 : Réduction des groupements thiols

4nmol d'ODN-thiol (oligonucléotides modifiés avec un ou plusieurs composés thiol) sont réduits dans une solution de Tris(2-carboxyethyl)phosphine hydrochlorure (TCEP, HCL, Sigma-Aldrich) (160mM) soit une concentration dans la solution de 20mM en TCEP,HCl, à 20°C, pendant 2h sous argon.

L'ODN est purifié par deux dilutions/centrifugations successives avec une solution de TCEP, HCl 20mM dégazée sur filtres amicon YM3000 (millipore) 15min à 14000rcf (rcf = Relative Centrifugal Force). Après dilution dans 450µL de tampon phosphate 100mM dégazé, le milieu est de nouveau centrifugé sur filtres amicon YM3000, 30 min, 14000rcf.

Une solution de greffage contenant 4nmol d'ODN, 90mM en phosphate de sodium, 2mM en TCEP, HCl est obtenue.

### Etape 2 : Activation de l'électrode d'or

L'électrode de travail d'or est nettoyée par un premier lavage dans l'acétone pendant 10 minutes aux ultra-sons. Une fois séchée la surface est plongée dans une solution « piranha » (H₂SO₄ 0,7mL, H₂O₂ 0,3mL) pendant 1 minute afin d'éliminer tout résidu organique de la surface.

Enfin l'activation basique de l'électrode consiste à nettoyer l'or en surface par production d'hydrogène à l'électrode par hydrolyse de l'eau dans la soude 0,5M dans les potentiels négatifs (-1,4V vs Ag/AgCl) pendant plusieurs cycles.

### Etape 3 : Greffage de la sonde

Après rinçage, la solution de greffage contenant l'oligonucléotide thiol est mise au contact de l'électrode d'or activée, pendant trois jours sous atmosphère inerte.

Après rinçage, la cellule électrochimique est remplie avec l'électrolyte d'analyse (phosphate de sodium dibasique 10mM, phosphate de potassium monobasique 10mM, perchlorate de sodium 250mM, pH 6,5).

La surface est passivée par une solution de mercaptopropanol 1mM pendant 30 minutes, après rinçage la cellule est mise dans l'électrolyte d'analyse pendant 2h afin de stabiliser la couche greffée.

### Etape 4 : Analyse de la stabilité des composés greffés en surface

Les analyses sont réalisées par voltampérométrie cyclique (CV) à 50mV/s ; entre -0,1V et 0,45V. Les études de stabilité de la couche greffée sont réalisées après une stabilisation du signal électrochimique pendant 2h en cyclant toutes les 30 minutes.

La cellule est remplie d'eau distillée dégazée à 60°C ou 80°C pendant 1 ou 5 minutes, après rinçage la cellule est remplie de 1,5mL d'électrolyte d'analyse phosphate (20mM) perchlorate (250mM). Après 30 minutes de stabilisation une CV est réalisée.

L'opération est renouvelée autant que nécessaire.

### Résultats

Une comparaison du taux de greffage des 3 oligonucléotides modifiés par des composés thiol (tétrathiol, dithiol et monothiol) a été réalisée. Le taux de greffage a été déterminé par intégration du pic d'oxydation du ferrocène. En effet, la charge d'électron transférée est directement liée au nombre de ferrocènes présents en surface de l'électrode, et donc au nombre de sondes greffées sur l'or.

Les résultats ci-dessous correspondent à la moyenne des taux de greffage de 3 greffages différents pour chaque sonde.

| | molécules/cm² |
|---|---|
| **monothiol** | 5,21E+12 |
| **dithiol** | 5,81E+12 |
| **tétrathiol** | 1,40E+12 |

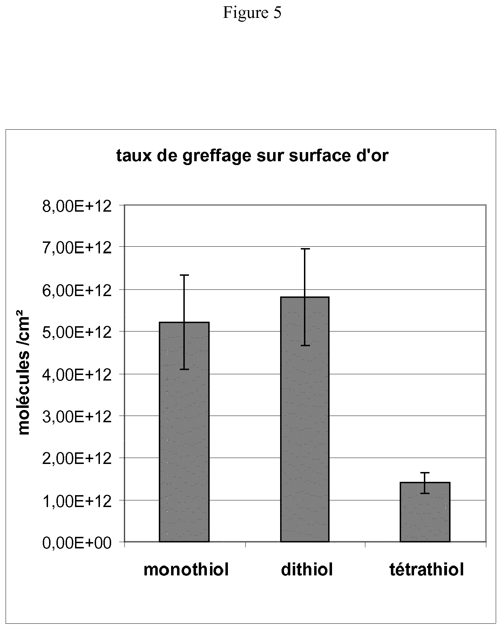

Un histogramme des résultats est représenté sur la figure 5.

Les taux de greffages pour le monothiol et dithiol sont assez comparables, nous pouvons observer un greffage moins important du tétrathiol probablement dû à l'encombrement plus important en accord avec le nombre de bras thiols. Malgré cette différence, le taux de greffage du tétrathiol reste important et il est très reproductible.

Une étude de stabilité des 3 oligonucléotides modifiés par des composés thiol vis-à-vis de la température a été menée dans de l'eau distillée dégazée. L'évolution de la réponse électrochimique a été suivie par voltampérométrie cyclique. Le pourcentage de diminution d'intensité d'oxydation du Ferrocène est calculé par rapport au signal obtenu après stabilisation.

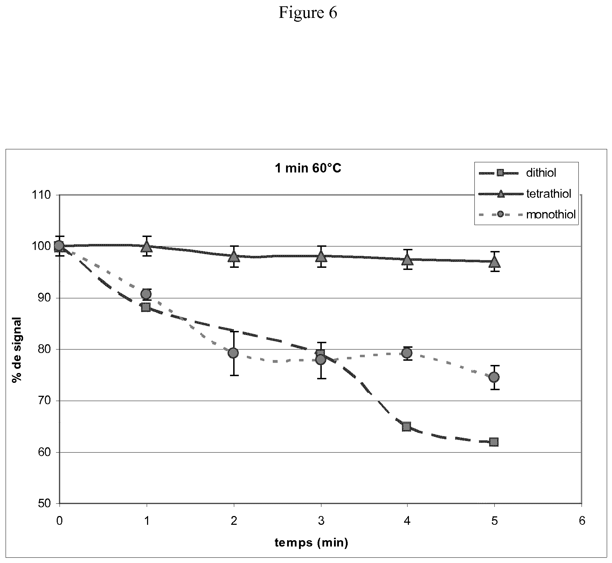

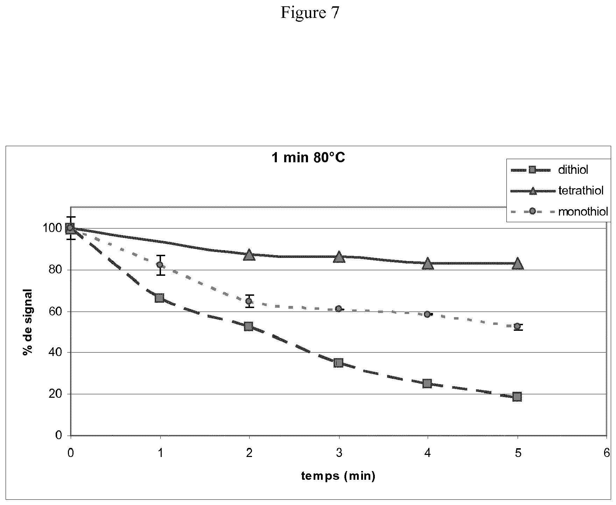

Ces diminutions en fonction du temps sont reportées sur les figures 6 et 7.

La molécule tétrathiol est très stable vis-à-vis de traitements successifs dans de l'eau à 60°C (figure 6). En effet, après 5 fois une minute de ce traitement, il reste 97% du signal de départ, contrairement au monothiol (70% de signal de départ) et dithiol (62%de signal de départ).

A 80°C, la perte de signal est plus importante (figure 7). Après cinq traitements successifs de une minute, 83% du signal de départ est encore quantifiable pour le tétrathiol. Ainsi, la stabilité du tétrathiol est bien supérieure à celle du monothiol (45% de signal résiduel) et celle du dithiol (18% de signal résiduel).

Cette étude montre le gain notable de stabilité du greffage thiol sur or par l'emploi d'un oligonucléotide modifié par 4 composés thiol (tétrathiol), en comparaison du greffage d'un monothiol ou d'un dithiol. Le manque de stabilité de ce dernier dithiol peut s'expliquer par une possible compétition entre le greffage sur or et la fermeture du cycle pour reformer le pont disulfure intramoléculaire. Il apparaît donc préférable de maintenir un nombre de quatre thiols sur le bras de greffage pour assurer une bonne stabilité vis-à-vis de la température.

### Application : détection de la présence du Virus de l'Hépatite C

### Echantillons cibles à tester

### 1) Cibles naturelles : amplicons VHC (+)

Prélèvement : les échantillons de plasmas issus de donneurs de sang dépistés positifs pour la présence du Virus de l'Hépatite C (VHC) à l'échelle nationale sont analysés par séquençage.

Amplification de l'ARN viral : Des amplicons VHC de 401pb sont produits dans la région virale NS5b par RT-PCR à partir des échantillons de plasmas décrits précédemment. L'ARN est extrait à partir de 200µL de plasma humain en utilisant le kit High Pure Viral Nucleic Acid (Roche) selon les recommandations du fabriquant. L'ARN est élué dans 50µL d'eau stérile (DNase/RNase free). Afin d'effectuer l'étape de rétro-transcription (RT), 11µL d'ARN sont dénaturés à 72°C pendant 10 minutes et rétro-transcrits en présence de 4µL de 5X First Strand Buffer (Invitrogen), 2µL de 10X Hexanucleotide Mix (Roche), 2µL de 10mM dNTP mix (Invitrogen) et 200U de SuperScript® II Reverse Transcriptase (Invitrogen). Les conditions de rétro-transcription sont les suivantes : 10 min à 23°C, 45 min à 37°C, et 10 min à 95°C. Cinq microlitres d'ADNc sont ensuite amplifiés par PCR (réaction en chaîne par polymérase) en utilisant les amorces « VHCsens biotinylée » (SEQ ID NO : 1 [Btn] Tgg ggA TCC CgT Atg ATA CCC gCT gCT TTg A) et « VHCanti-sens » (SEQ ID NO : 2 ggC ggA ATT CCT ggT CAT AgC CTC CgT gAA) (voir Catherine Tamalet, Philippe Colson, Hervé Tissot-Dupont, Mireille Henry, Christian Tourres, Natacha Tivoli, Danielle Botta, Isabelle Ravaux, Isabelle Poizot-Martin and Nouara Yahi. 2003, Journal of Medical Virology 71:391-398) dans 50µL de mélange réactionnel : 1X PCR Buffer sans MgCl2 (Invitrogen), 0,2µM de chaque amorce, 1,5mM MgCl2 (Invitrogen), 0,2mM dNTP mix (Invitrogen) et 1,3U Taq Polymerase (Invitrogen). Les conditions de PCR sont les suivantes : 5 min à 95°C, 40 cycles (dénaturation: 40 sec, 95°C; hybridation: 40 sec, 56°C; élongation: 50 sec, 72°C), et une extension finale de 10 min à 72°C.

Les amplicons VHC obtenus sont analysés par électrophorèse en gel d'agarose, aliquotés et conservés à -20°C avant utilisation.

### 2) Cibles synthétiques : oligonucléotides biotinylés

Des oligonucléotides de 15-mères biotinylés à l'extrémité 5' ont été synthétisés. Ces oligonucléotides sont strictement complémentaires aux sondes VHC choisies comme suit.

### Design des sondes oligonucléotidiques pour la reconnaissance de séquences VHC.

La région NS5b du VHC a été ciblée pour le design des sondes oligonucléotidiques.

Les séquences des génomes VHC amplifiés dans la région NS5b (banque de 800 échantillons) ont été analysées à l'aide des logiciels d'alignement clustalW2 et de phylogénie Mega5 afin d'identifier les zones les plus conservées. Une région très conservée permettant l'amplification spécifique de tous les génomes de génotype viral 1a1b a été sélectionnée ainsi qu'une seconde région permettant l'amplification spécifique des génomes de génotype viral 3a. Deux sondes de 15-mères ont été dessinées complémentaires de chacune de ces régions. Le design des sondes 1a1b et 3a est alors utilisé pour la synthèse des oligonucléotides multi-thiols.

### Evaluation des hybridations sondes l cibles par ELOSA (Enzyme-Linked OligoSorbent Assay)

Le greffage de tout type de sondes thiols (oligonucléotides d'anomérie alpha ou béta, sondes linéaires ou structurées (par exemple tige-boucle) peut être réalisé selon ce protocole optimisé après lavage des puits de microplaques activées maléimide (Pierce) par le tampon WB1 (0,1M Na2HPO4, 0,15M NaCl, 0,05% Tween 20 (w/v), pH 7,2). La fonctionnalisation des puits est réalisée avec 100nM de sondes multi-thiols dans du tampon BB (0,1M Na2HPO4, 0,15M NaCl, 10mM EDTA, pH 7,2) pendant 2 heures à température ambiante (TA). Les puits sont ensuite lavés trois fois avec WB1, saturés par une solution de Cystéine-HCl 10 µg.mL⁻¹ dans BB (Pierce) pendant 1 heure à TA, et lavés à nouveau trois fois avec WB1.

Les tests d'hybridation peuvent être réalisés avec les cibles synthétiques courtes 15-mères ou avec de réels amplicons VHC longs (401 nucléotides) décrits plus haut. Les cibles synthétiques et les amplicons sont dilués dans 150 µL de tampon d'hybridation (TH : 0,9M NaCl, 60mM NaH2PO4, 6mM EDTA, pH 7,4, Denhardt 5X) avant d'être déposés dans les puits. Une étape supplémentaire de dénaturation de 10 min à 95°C est effectuée sur les amplicons avant transfert dans les micropuits. L'hybridation est réalisée sur la nuit à 37°C. Les puits sont lavés avec du tampon WB2 (0,75M NaCl, 50mM NaH2PO4, 5mM EDTA, pH 7,4, SDS 0,1 %) trois fois 2 min à TA et une fois 30 min à 50°C.

L'étape de détection est réalisée après incubation pendant 30 min à TA des puits en présence de 100 µL/puits de Streptavidine-Europium diluée dans 100 µL de tampon de test (« Assay Buffer », Perkin Elmer). Les puits sont finalement lavés six fois avec du tampon WB3 (WB1X, Perkin Elmer) et 200 µL de tampon d'amplification (« Enhancement Buffer », Perkin Elmer) sont ajoutés dans chaque puits pendant 5 min à TA. La fluorescence en temps résolu est mesurée sur un détecteur multi-marquage Victor3TM 1420 (« multilabel counter », Perkin Elmer) selon le protocole du fabricant (excitation à 340 nm et émission à 615 nm).

Le test ELOSA est réalisé avec des amplicons spécifiques du génotype VHC 3a (dilutions 1/10, 1/100 et 1/1000) et des amplicons spécifiques du génotype VHC 1a1b (et donc non spécifiques du génotype 3a). Le contrôle d'hybridation est effectué avec des cibles synthétiques (15-mères) spécifiques du génotype 3a (complémentaire de la sonde), testées à 1000 pM et 5 pM et des cibles synthétiques spécifiques du génotype 1a1b, testées à 1000 pM.

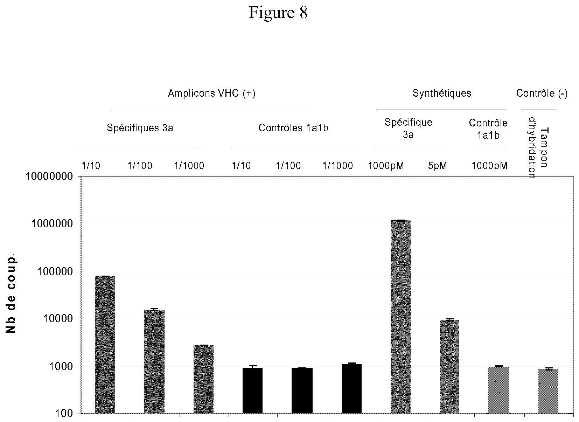

Le graphique de la figure 8, correspondant au test de fluorescence, illustre les résultats du test ELOSA et quantifie l'hybridation avec la sonde tétrathiol correspondant au génotype VHC 3a. Les tests « contrôle 1a1b » correspondent au bruit de fond ; les résultats « spécifiques 3a » doivent donc dépasser le bruit de fond pour que le test permette de quantifier l'hybridation.

### Etude de l'effet de la densité de greffage

Le même test ELOSA est effectué en modifiant la concentration de la solution de sondes (monothiol, dithiol et tétrathiol) pour la faire varier de 1 nM à 100 nM. Le test ELOSA est effectué avec des cibles 1a1b et 3a à courtes chaînes (15-mères) et à chaînes moyennes (105-mères). Les tests effectués avec les sondes spécifiques du génotype 1a1b montrent une très bonne spécificité d'hybridation avec les cibles spécifique 1a1b et aucune hybridation avec des cibles non complémentaires de génotype 3a.

La détection de l'hybridation sonde/cible s'effectue par fluorescence comme précédemment (figure 8).

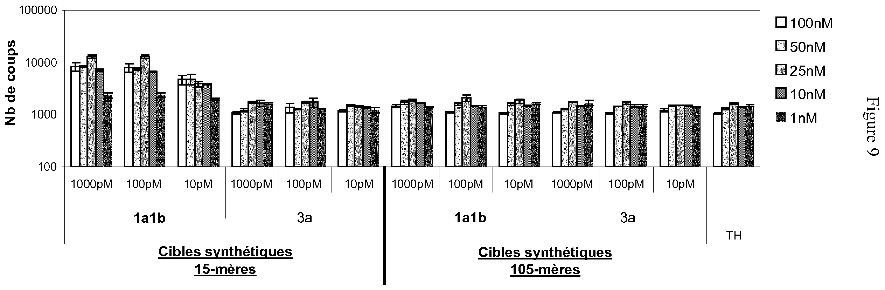

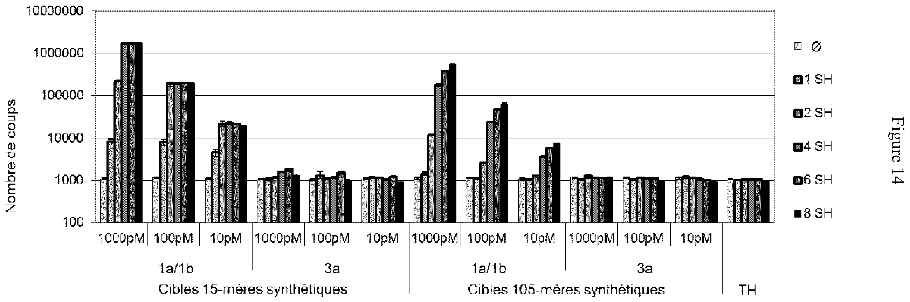

Les résultats pour chaque type de sondes (monothiol, dithiol et tétrathiol) sont représentés sur les graphiques des figures 9, 10 et 11. Le contrôle TH correspond à un test effectué dans les puits mais sans la présence des sondes. D'autres sondes (comprenant 6 et 8 thiols) ont également été testées et sont représentées sur la figure 14.

La figure 9 illustre les résultats obtenus pour la sonde monothiol, c'est-à-dire comprenant un seul composé thiol selon l'invention. La sonde monothiol n'est pas efficace pour la détection de cible 105-mères, le signal pour les cibles 105-mères étant identique au signal de contrôle TH. Par ailleurs, la figure 9 montre également que la concentration de sonde utilisée pour le greffage entre 10 nM et 100 nM n'a aucun effet sur l'efficacité de l'hybridation, en effet, il n'y a pas de différence significative entre le test effectué avec 10 nM de sondes et celui effectué avec 100 nM de sondes. Par contre l'utilisation d'une concentration de 1 nM en sonde amène une baisse de l'efficacité de l'hybridation.

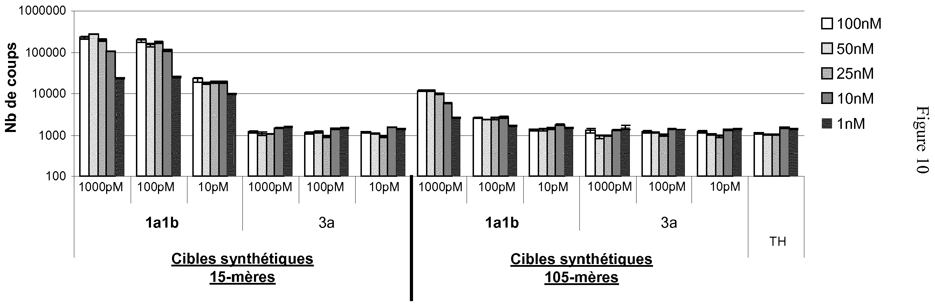

La figure 10 illustre les résultats obtenus pour la sonde dithiol, c'est-à-dire comprenant deux composés thiol selon l'invention. La sonde dithiol se révèle peu efficace pour la détection de cibles 105-mères, une détection de l'hybridation étant observée pour une concentration de cibles 1a1b de 1000 pM. Par ailleurs, la figure 10 montre que la densité de greffage influe légèrement sur la détection de l'hybridation. En effet, le signal de fluorescence est plus important lorsque la concentration de sondes passe de 1 nM à 100 nM avec peu de différence entre 25 nM et 100 nM.

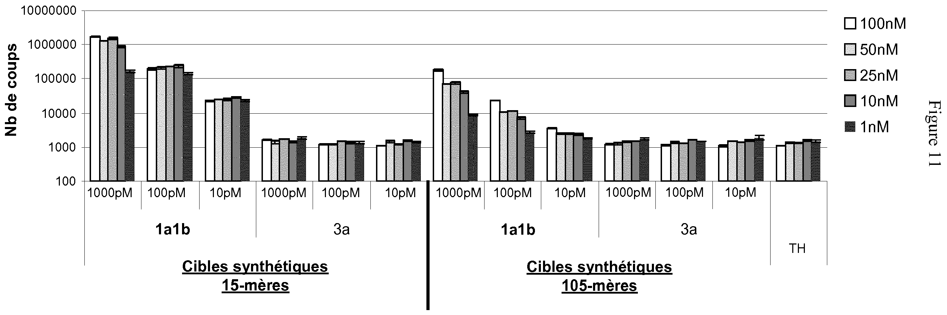

La figure 11 illustre les résultats obtenus pour la sonde tétrathiol, c'est-à-dire comprenant quatre composés thiol selon l'invention. La sonde tétrathiol se révèle très efficace pour la détection de cibles 105-mères, le signal de fluorescence est bien supérieur au signal de fluorescence du contrôle TH, en particulier pour une concentration de cibles de 100 pM et 1000 pM. Par ailleurs, la figure 11 montre que la densité de greffage influe sur la détection de l'hybridation pour les 105-mères. En effet, pour une même concentration de cibles, par exemple 1000 pM, le signal de fluorescence est plus important lorsque la concentration de sondes est de 100 nM, par rapport à une concentration de sondes de 1 nM. Cet effet, aussi nommé « dose dépendance » est très marqué pour la détection de cibles 105-mères.

La figure 14 montre que des sondes comportant 6 ou 8 thiol donnent des résultats d'hybridation satisfaisants. En effet, le signal de fluorescence pour ces deux sondes est similaire à celui de la sonde tétrathiol.

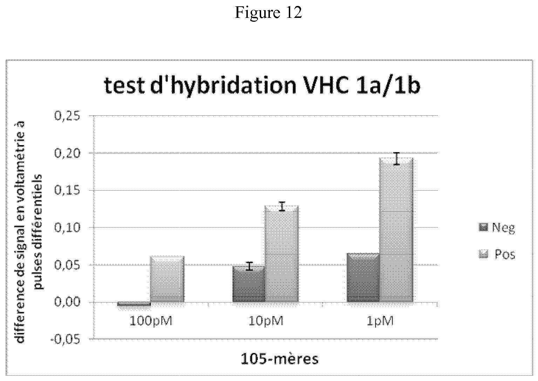

La figure 12 illustre les résultats d'un test d'hybridation mené avec la séquence permettant le génotypage 1a/1b de HCV. Le test est mené à trois concentrations en cible, 100 pM, 10pM et 1pM, avec des cibles synthétiques de 105 bases de longueur. Le contrôle négatif est mené avec la cible 3a non complémentaire. La sonde tétrathiol 1a/1b est greffée sur l'électrode de travail à surface or de la cellule électrochimique. La réaction de reconnaissance est suivie par voltamétrie à pulses différentiels. Les valeurs indiquées en ordonnée correspondent aux valeurs normalisées de variation de courant. Ce test par électrochimie est sensible et spécifique à une concentration en cible de 1pM. La variation de signal apparaît être plus forte que pour le test mené à 100pM. A plus forte concentration en cible, un effet d'adsorption non spécifique des cibles sur la surface de l'électrode réduit l'efficacité de la réaction de reconnaissance. La variation de signal du test mené à 100pM apparaît plus faible que celle observée pour le test à 1pM. Cette méthode électrochimique ne permet pas un suivi quantitatif de la réaction d'hybridation. Néanmoins, elle fournit une réponse oui/non spécifique d'une très grande sensibilité.

## Revendications

1. Composé répondant à la formule (I) suivante : 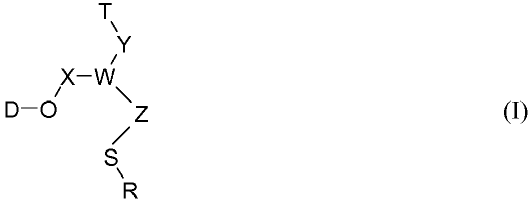 dans laquelle :
T est un groupement choisi parmi -O-P(OR₁)N(R₂)₂, -O-PH(O)O⁻, -OC(O)JC(O)NH-□,
∘ R₁ est choisi parmi les groupements 2-cyanoéthyle, R'₁R'₂R'₃SiCH₂CH₂, et R'₁, R'₂, R'₃ identiques ou différents représentent un groupement choisi parmi les alkyles linéaires ou ramifiés comportant de 1 à 12 atomes de carbone et les aryles en C6-C12,
∘ R₂ est choisi parmi les groupements alkyles linéaires ou ramifiés comportant de 1 à 12 atomes de carbone, pyrrolidine,
∘ J est choisi parmi une simple liaison, un groupement -CH₂-, -CH₂CH₂-, -CH₂OCH₂-, -CH₂OPhOCH₂- où Ph est un benzyle,
∘ □ représente un support solide,
D est un groupement protecteur des alcools choisi parmi le 4,4'-diméthoxytrityle, le 9-phenylxanthèn-9-yl, le fluorenylmethyloxycarbonyl et le tert-butyl-diméthylsilyl,
W est choisi parmi les groupements CH, CCH₃, CCH₂CH₃, cyclohexanes tri-yle, et benzènes triyle,
Z est choisi parmi les groupements alkoxy en C1-C12, cyclohétéroalkyles oxygéné ou azoté en C3-C12, NCO-alkyles en C1-C12, CON-alkyles en C1-C12,
Y est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
X est choisi parmi les groupements alkyles linéaires ou ramifiés en C1-C12, aminoalkyles en C1-C12, alkoxy en C1-C12, cycloalkyles en C3-C12, cyclohétéroalkyles oxygénés ou azotés en C3-C12,
R est choisi parmi les groupements acyles en C1-C12, S-alkyles en C1-C12, S-aryles en C6-C12, S-2-pyridine, S-hétéroalkyles oxygénés ou azotés en C1-C12, S-cycloalkyles en C3-C12, S-cyclohétéroalkyles oxygénés ou azotés en C3-C12.

2. Composé selon la revendication 1 répondant à l'une des formules (Ia), (Ib) et (Ic) ci-dessous :

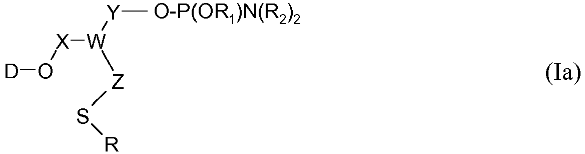

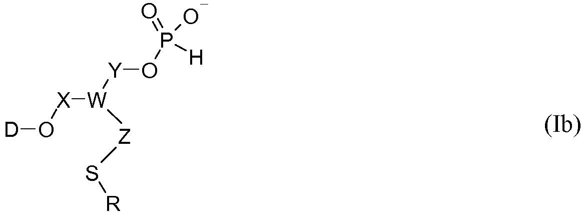

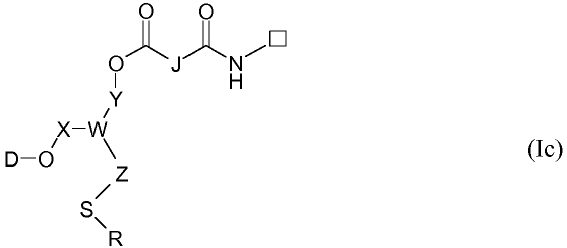

3. Composé selon la revendication 1 ou la revendication 2, dans lequel :
• D est choisi parmi le 4,4'-diméthoxytrityle, le 9-phenylxanthen-9-yl ou le Fluorenylmethyloxycarbonyl,
• W est un groupement choisi parmi CH, CCH₃, CCH₂CH₃, cyclohexane tri-yle et benzène tri-yle ; et/ou
• Z est choisi parmi les groupements alkoxy en C1-C12, cyclohétéroalkyles oxygéné ou azoté en C3-C12, NCO-alkyles en C1-C12, CON-alkyles en C1-C12 ; et/ou
• Y est choisi parmi les groupements alkyles linéaires ou ramifiés en C1-C6, aminoalkyles en C1-C6, alkoxy en C1-C6, cycloalkyles en C3-C6, cyclohétéroalkyles oxygénés ou azotés en C3-C6 ; et/ou
• X est choisi parmi les groupements alkyles linéaires ou ramifiés en C1-C6, aminoalkyles en C1-C6, alkoxy en C1-C6, cycloalkyles en C3-C6, cyclohétéroalkyles oxygénés ou azotés en C3-C6 ; et/ou
• R est choisi parmi les groupements acyles en C1-C6, S-alkyles en C1-C6, S-aryles en C6-C6, S-hétéroalkyles oxygénés ou azotés en C1-C6, S-cycloalkyles en C3-C6, S-cyclohétéroalkyles oxygénés ou azotés en C3-C6, de préférence R est un acyle en C1-C6.

4. Composé selon l'une quelconque des revendications 1 à 3, dans lequel le groupement T est un groupement -O-P(OR₁)N(R₂)₂ où R₂ est un groupement isopropyle et R₁ est choisi parmi les groupements 2-cyanoéthyle, 2-(triméthylsilyl)éthyle, 2-(triphénylsilyl)éthyle, 2-(diphénylméthylsilyl)éthyle.

5. Composé selon l'une quelconque des revendications 1 à 3, dans lequel le groupement T est le groupement -OC(O)JC(O)NH-□ où □ est un support solide choisi parmi les résines, en particulier parmi les résines à base de polystyrène, polyacrylamide, polyéthylèneglycol, cellulose, polyéthylène, polyester, latex, polyamide, polydiméthylacrylamide, les polymères hydrophiles synthétiques ou naturels, les billes de verre, les gels de silice.

6. Procédé de fabrication d'un composé de formule (I) selon l'une quelconque des revendications 1 à 5 comprenant au moins une étape choisie parmi les étapes suivantes :
- préparation du composé (Ia) à partir du composé (II) selon le schéma de synthèse suivant : 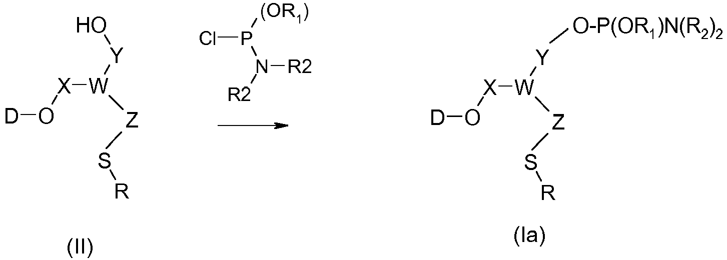 ou selon le schéma de synthèse suivant : 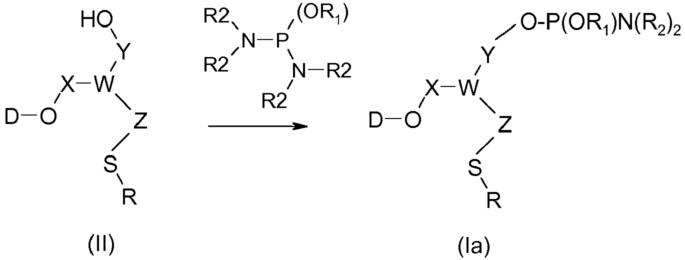
- préparation du composé (Ib) à partir du composé (II) selon le schéma de synthèse suivant : 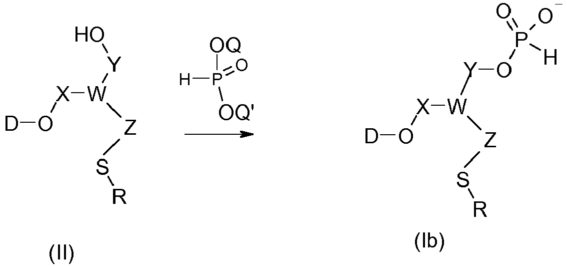 dans laquelle Q et Q' représentent, indépendamment l'un de l'autre, un groupement benzène substitué ou non,
- préparation du composé (Ic) à partir du composé (II) selon le schéma de synthèse suivant : 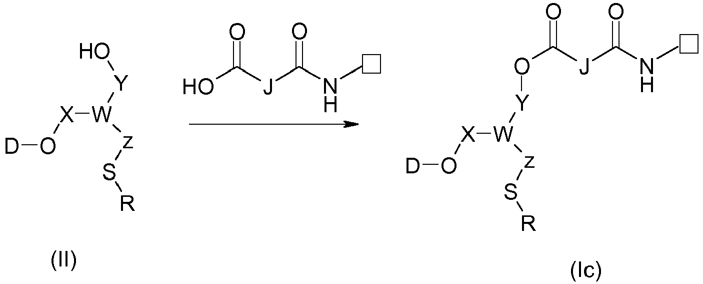 ou selon le schéma de synthèse suivant : 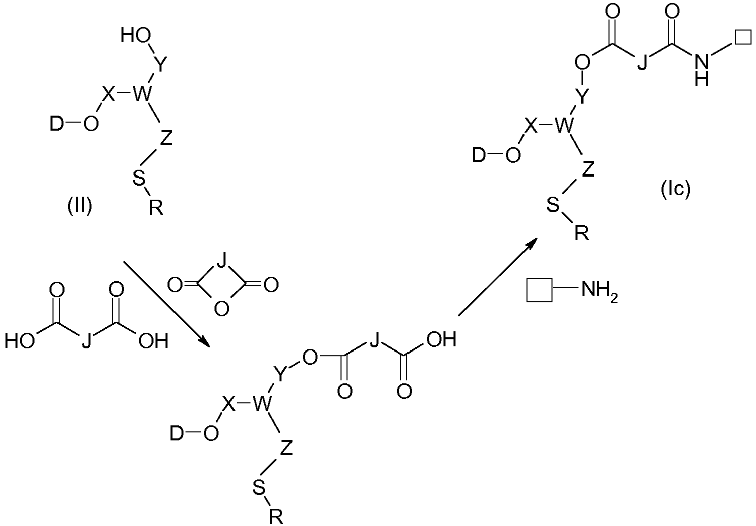 D, X, Y, Z, W, R, □, R₁ et R₂ ayant la même définition dans chacune de ces étapes que dans les revendications 1 à 5.

7. Oligomère susceptible d'être obtenu par oligomérisation d'un composé (I) selon l'une des revendications 1 à 5, répondant à la formule
(Ic) - (Δ)ₖ (XVI)ₖ
dans laquelle :
(Ic) a la même signification que dans les revendications 2 à 5, le groupement R du composé (Ic) peut en outre représenter H.
k représente un entier compris entre 1 et 12,
Δ représente (I'a) ou (I'b) où 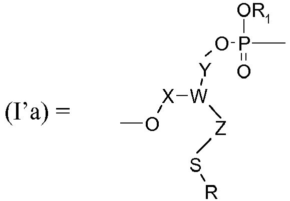 et 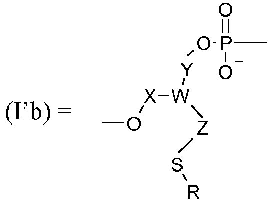 dans lesquelles, X, Y, W, Z, R, R₁ ont la même définition que dans les revendications 1 à 5, R peut en outre représenter H.

8. Oligomère selon la revendication 7, répondant à la formule (XIV)ₖ : 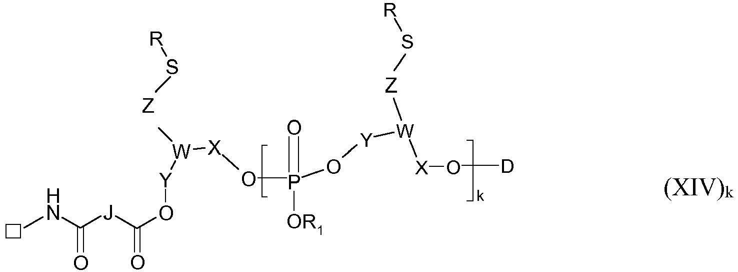 dans laquelle,
□, D, X, Y, W, Z, R et R₁ ont la même définition que dans les revendications 1 à 4, R peut en outre représenter H et D peut en outre représenter H,
k est un nombre entier compris entre 1 et 11.

9. Procédé de préparation d'un oligonucléotide modifié comprenant au moins :
- une étape de greffage d'un composé (I) selon l'une quelconque des revendications 1 à 5 sur un oligonucléotide, ou
- une étape de greffage d'un nucléotide sur un oligomère selon la revendication 7 ou la revendication 8.

10. Oligonucléotide modifié susceptible d'être obtenu par le procédé selon la revendication 9 répondant à la formule (XIIa) suivante :
□ - N₁ - N₂- ...- Nₙ₋₁- Nₙ- (I')_{y} -(M₁ -...- Mₘ₋₁- Mₘ)ₚ - (I')_{y}
dans laquelle,
N₁, ...Nₙ représentent indépendamment les uns des autres un nucléotide,
M₁, ...Mₘ représentent indépendamment les uns des autres un nucléotide,
(I') représente un composé de formule (I'a) ou (I'b) tel que définis à la revendication 7,
n est un nombre entier compris entre 1 et 100,
m est un nombre entier compris entre 1 et 100,
y est un nombre entier compris entre 1 et 12,
p représente 0 ou 1,
y' est un nombre entier compris entre 0 et 12 si p vaut 1 et, y' est égal à 0 si p vaut 0,
□ représente un support solide.

11. Oligonucléotide modifié susceptible d'être obtenu par le procédé selon la revendication 9 répondant à la formule (XIIIa) suivante :
(Ic) - (I')_{y-1}- N₁ - N₂- ...- Nₙ₋₁- Nₙ - (I')_{y} -[M₁ - M₂ - ... - Mₘ₋₁- Mₘ]ₚ-(I')_{y}"
dans laquelle,
N₁, ...Nₙ représentent indépendamment les uns des autres un nucléotide,
M₁, ... Mₘ représentent indépendamment les uns des autres un nucléotide,
(I') représente un composé de formule (I'a) ou (I'b) tel que définis à la revendication 7,
n est un nombre entier compris entre 1 et 100,
m est un nombre entier compris entre 1 et 100,
y est un nombre entier compris entre 1 et 12,
y' est un nombre entier compris entre 0 et 12,
p vaut 0 ou 1 si y' est différent de 0 et, si y' vaut 0 alors p vaut 0,
y" est un nombre entier compris entre 0 et 12 si p vaut 1 et, si p vaut 0 alors y" vaut 0.

12. Oligonucléotide modifié selon la revendication 10 répondant à la formule (XIIc) : 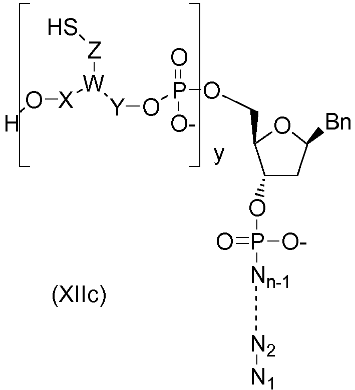 dans laquelle,
n, y, N₁, ...Nₙ₋₁ ont la même définition que dans la revendication 8,
X, Y, Z, W ont la même définition que dans les revendications 1 à 4,
Bn représente la base du n-ième nucléotide.

13. Oligonucléotide modifié selon la revendication 11 répondant à la formule (XIIIc) : 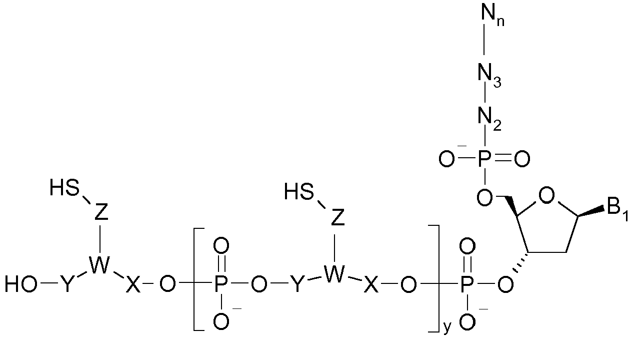 dans laquelle,
n, y, N ont la même définition que dans la revendication 9,
X, Y, Z, W ont la même définition que dans les revendications 1 à 4,
B₁ représente la base du 1er nucléotide.

14. Dispositif automatisé pour la synthèse de nucléotides comportant au moins :
• des réservoirs distincts contenant :
- des nucléotides,
- des activateurs de couplage et
- des produits de lavage,
• des moyens mécaniques de prélèvement et de distribution d'échantillons de produits, ainsi que des moyens informatiques permettant la mise en oeuvre contrôlée de ces moyens mécaniques, ainsi que :
• au moins un réservoir dans lequel est placé un support solide greffé par un oligomère selon la revendication 7 ou 8, et/ou au moins un réservoir contenant un composé (Ia) ou un composé (Ib) selon la revendication 2.

15. Substrat greffé par au moins un oligonucléotide modifié selon l'une des revendications 10 à 13, ledit substrat comportant au moins une zone de réception revêtue d'un film d'or ou de platine ou greffée par des groupements comportant au moins une double liaison carbone-carbone ou une triple liaison carbone-carbone ou des groupements halogénoacétamide, de préférence des groupements maléimide et/ou acrylamide, ledit substrat étant en métal dans le cas du film d'or et en plastique dans le cas du greffage par des groupements comportant au moins une double liaison carbone-carbone ou une triple liaison carbone-carbone ou des groupements halogénoacétamide, de préférence des groupements maléimide et/ou acrylamide.

16. Substrat selon la revendication précédente dans lequel le substrat en métal est en cuivre ou en titane et/ou le substrat en plastique est en polystyrène.

17. Kit de test comprenant :
- au moins un substrat comportant au moins une zone de réception revêtue d'un film métallique d'or ou de platine ou d'un substrat greffé par des groupements comportant au moins une double liaison carbone-carbone ou une triple liaison carbone-carbone ou des groupements halogénoacétamide, de préférence des groupements maléimide et/ou acrylamide,
- au moins un oligonucléotide modifié selon l'une des revendications 10 à 13.

18. Utilisation d'un substrat selon la revendication 15 pour réaliser des tests d'affinité entre un oligonucléotide et une autre molécule.

## Patentansprüche

1. Verbindung, die der folgenden Formel (I) entspricht: 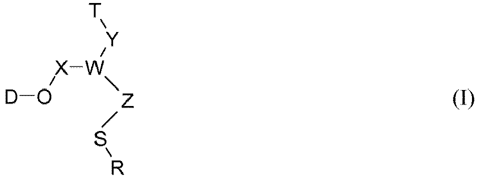 wobei:
T eine Gruppierung ist, die unter -O-P(OR₁)N(R₂)₂, -O-PH(O)O, -OC(O)JC(O)NH-□ ausgewählt ist,
* R₁ ausgewählt ist unter den Gruppierungen 2-Cyanethyl, R'₁R'₂R'₃SiCH₂CH und R'₁, R'₂, R'₃ identisch oder unterschiedlich sind und eine Gruppierung darstellen, die ausgewählt ist unter den linearen oder verzweigten Alkylen, die 1 bis 12 Kohlenstoffatome, und das C6-C12-Aryl umfassen,
* R₂ ausgewählt ist unter den linearen oder verzweigten Alkylgruppierungen, die 1 bis 12 Kohlenstoffatome, Pyrrolidin umfassen,
* J ausgewählt ist unter einer einfachen Verbindung, einer Gruppierung -CH₂-, -CH₂CH₂-,-CH₂OCH₂-, -CH₂OPhOCH₂-, wobei Ph ein Benzyl ist,
* □ einen festen Träger darstellt,
D eine Schutzgruppierung der Alkohole ist, die ausgewählt ist unter 4,4'-Dimethoxytrityl, 9-Phenylxanthen-9-yl, Fluorenylmethyloxycarbonyl und Tert-Butyl-Dimethylsilyl,
W unter den Gruppierungen von CH, CCH₃, CCH₂CH₃, Triyl-Cyclohexanen und Triyl-Benzolen, ausgewählt ist
Z unter den Gruppierungen von C1-C12-Alkoxy, sauerstoffhaltigen oder stickstoffhaltigen C3-C12-Cycloheteroalkylen, C1-C12-NCO-Alkylen, C1-C12-CON-Alkylen ausgewählt ist,
Y unter den linearen oder verzweigten Gruppierungen von C1-C12-Alkylen, C1-C12-Aminoalkylen, C1-C12-Alkoxy, C3-C12-Cycloalkylen, sauerstoffhaltigen oder stickstoffhaltigen C3-C12-Cycloheteroalkylen ausgewählt ist,
X unter den linearen oder verzweigten Gruppierungen von C1-C12-Alkylen, C1-C12-Aminoalkylen, C1-C12-Alkoxy, C3-C12-Cycloalkylen, sauerstoffhaltigen oder stickstoffhaltigen C3-C12-Cycloheteroalkylen ausgewählt ist,
R unter den Gruppierungen von C1-C12-Acylen, C1-C12-S-Alkylen, C6-C12-S-Arylen, S-2-Pyridin, sauerstoffhaltigen oder stickstoffhaltigen C1-C12-S-Heteroalkylen, C3-C12-S-Cycloalkylen, sauerstoffhaltigen oder stickstoffhaltigen C3-C12-S-Cycloheteroalkylen ausgewählt ist.

2. Verbindung nach Anspruch 1, die einer der unteren Formeln (Ia), (Ib) und (Ic) entspricht:

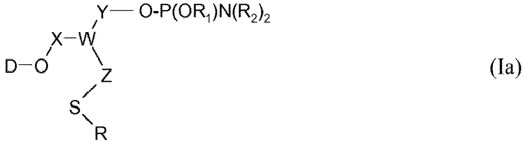

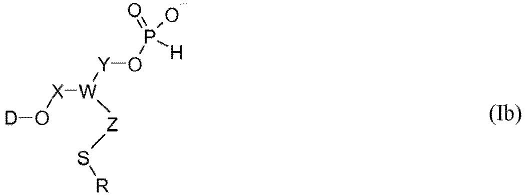

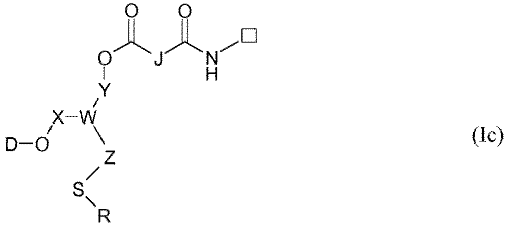

3. Verbindung nach Anspruch 1 oder Anspruch 2, bei der:
* D unter 4,4`-Dimethoxytrityl, 9-Phenylxanthen-9-yl oder Fluorenylmethyloxycarbonyl ausgewählt ist,
* W unter den Gruppierungen von CH, CCH₃, CCH₂CH₃, Triyl-Cyclohexan und Triyl-Benzol ausgewählt ist, und/oder
* Z unter den Gruppierungen von C1-C12-Alkoxy, sauerstoffhaltigen oder stickstoffhaltigen C3-C12- Cycloheteroalkylen, C1-C12-NCO-Alkylen, C1-C12-CON-Alkylen ausgewählt ist, und/oder
* Y unter den linearen oder verzweigten Gruppierungen von C1-C6-Alkylen, C1-C6-Aminoalkylen, C1-C6-Alkoxy, C3-C6-Cycloalkylen, sauerstoffhaltigen oder stickstoffhaltigen C3-C6-Cycloheteroalkylen ausgewählt ist, und/oder
* X unter den linearen oder verzweigten Gruppierungen von C1-C6-Alkylen, C1-C6-Aminoalkylen, C1-C6-Alkoxy, C3-C6-Cycloalkylen, sauerstoffhaltigen oder stickstoffhaltigen C3-C6-Cycloheteroalkylen ausgewählt ist, und/oder
* R ausgewählt ist unter den Gruppierungen von C1-C6-Acylen, C1-C6-S-Alkylen, C6-C6-S-Arylen, sauerstoffhaltigen oder stickstoffhaltigen C1-C6-S-Heteroalkylen, C3-C6-S-Cycloalkylen, sauerstoffhaltigen oder stickstoffhaltigen C3-C6-S-Cycloheteroalkylen, wobei R vorzugsweise ein C1-C6-Acyl ist.

4. Verbindung nach einem der Ansprüche 1 bis 3, bei der die T-Gruppierung eine Gruppierung -O-P(OR₁)N(R₂)₂ ist, wobei R₂ eine Isopropylgruppierung ist, und R₁ unter den Gruppierungen 2-Cyanethyl, 2-(Trimethylsilyl)ethyl, 2-(Triphenylsilyl)ethyl, 2-(Diphenylmethylsilyl)ethyl ausgewählt ist.

5. Verbindung nach einem der Ansprüche 1 bis 3, bei der die T-Gruppierung die Gruppierung -OC(O)JC(O)NH-O ist, wobei □ ein fester Träger ist, der ausgewählt ist unter den Harzen, insbesondere unter den Harzen auf Basis von Polystyrol, Polyacrylamid, Polyethylenglycol, Zellulose, Polyethylen, Polyester, Latex, Polyamid, Polydimethylacrylamid, den synthetischen oder natürlichen hydrophilen Polymeren, den Glaskugeln, den Kieselgelen.

6. Verfahren zur Herstellung einer Verbindung der Formel (I) nach einem der Ansprüche 1 bis 5, welche mindestens einen Schritt umfasst, der unter den folgenden Schritten ausgewählt ist:
- Herstellung der Verbindung (Ia) aus der Verbindung (II) nach dem folgenden Syntheseschema: 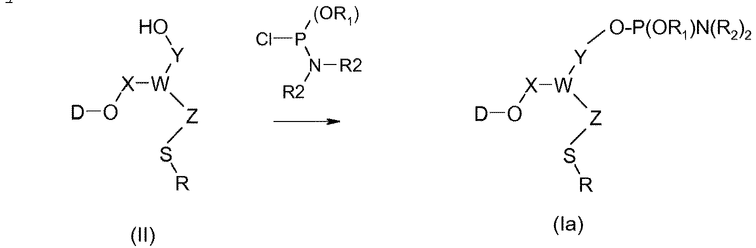 oder nach dem folgenden Syntheseschema: 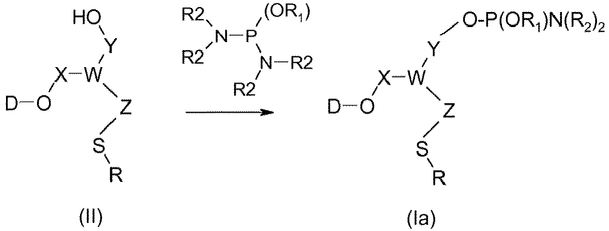
- Herstellung der Verbindung (Ib) aus der Verbindung II) nach dem folgenden Syntheseschema: 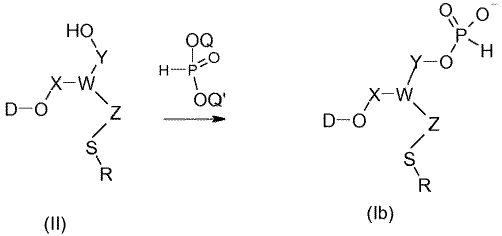 wobei Q und Q' unabhängig voneinander eine Benzolgruppierung darstellen, die substituiert ist oder nicht,
- Herstellung der Verbindung (Ic) aus der Verbindung (II) nach dem folgenden Syntheseschema: 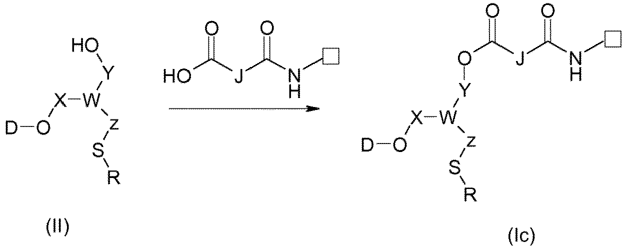 oder nach dem folgenden Syntheseschema: 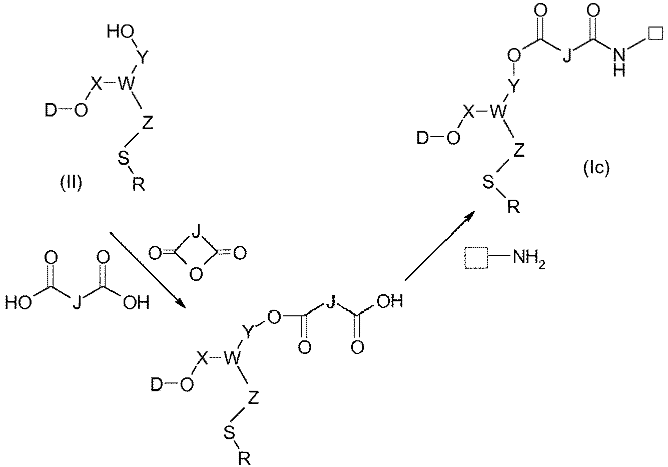 wobei D, X, Y, Z, W, R, □, R₁ und R₂ in jedem dieser Schritte dieselbe Definition haben wie in den Ansprüchen 1 bis 5.

7. Oligomer, das durch Oligomerisation einer Verbindung (I) nach einem der Ansprüche 1 bis 5 erhalten werden kann, was folgender Formel entspricht:
(Ic) - (Δ)ₖ (XVI) ₖ
wobei:
(Ic) dieselbe Bedeutung wie in den Ansprüchen 2 bis 5 hat, wobei die R-Gruppierung der Verbindung (Ic) ferner H darstellen kann,
k eine ganze Zahl zwischen 1 und 12 darstellt,
Δ (I'a) oder (I'b) darstellt, wobei: 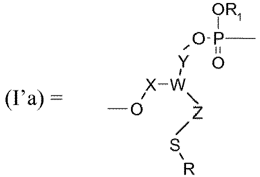 und 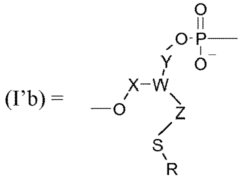 wobei X, Y, W, Z, R, R₁ dieselbe Definition wie in den Ansprüchen 1 bis 5 haben, wobei R ferner H darstellen kann.

8. Oligomer nach Anspruch 7, das der Formel (XIV)ₖ entspricht: 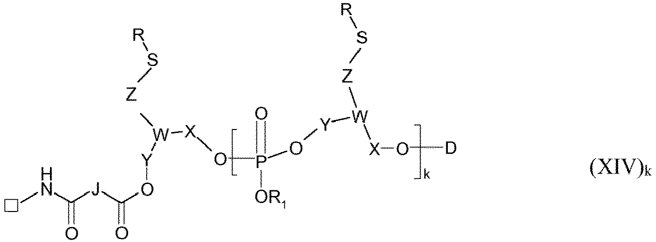 Wobei,
□, D, X, Y, W, Z, R und R₁ dieselbe Definition wie in den Ansprüchen 1 bis 4 haben, wobei R ferner H darstellen kann, und D ferner H darstellen kann,
k eine ganze Zahl zwischen 1 und 11 ist.

9. Verfahren zur Herstellung eines modifizierten Oligonukleotids, welches mindestens umfasst:
- einen Schritt des Pfropfens einer Verbindung (I) nach einem der Ansprüche 1 bis 5 auf ein Oligonukleotid, oder
- einen Schritt des Pfropfens eines Nukleotids auf ein Oligomer nach Anspruch 7 oder Anspruch 8.

10. Modifiziertes Oligonukleotid, das durch das Verfahren nach Anspruch 9 erhalten werden kann, und das der folgenden Formel (XIIa) entspricht:
□ - N₁-N₂- ...- Nₙ₋₁-Nₙ-(I')_{y}-(M₁ -...- Mₘ₋₁-Mₘ)ₚ-(I')_{y'}
wobei,
N₁, ...Nₙ unabhängig voneinander ein Nukleotid darstellen,
M₁, ...Mₘ unabhängig voneinander ein Nukleotid darstellen,
(I`) eine Verbindung der Formel (I'a) oder (I'b), wie in Anspruch 7 definiert, darstellt,
n eine ganze Zahl zwischen 1 und 100 ist,
m eine ganze Zahl zwischen 1 und 100 ist,
y eine ganze Zahl zwischen 1 und 12 ist,
p 0 oder 1 darstellt,
y' eine ganze Zahl zwischen 0 und 12 ist wenn p gleich 1 ist, und y' gleich 0 ist wenn p gleich 0 ist,
□ einen festen Träger darstellt.

11. Modifiziertes Oligonukleotid, das durch das Verfahren nach Anspruch 9 erhalten werden kann, und das der folgenden Formel (XIIIa) entspricht:
(Ic) - (I')_{y-1} - N₁ - N₂- ...- Nₙ₋₁ - Nₙ - (I')_{y'} - [M₁ - M₂ - ... - Mₘ₋₁- Mₘ]ₚ-(I')_{y"}
wobei,
N₁, ...Nₙ unabhängig voneinander ein Nukleotid darstellen,
M₁, ...Mₘ unabhängig voneinander ein Nukleotid darstellen,
(I') eine Verbindung der Formel (I'a) oder (I'b), wie in Anspruch 7 definiert, darstellt,
n eine ganze Zahl zwischen 1 und 100 ist,
m eine ganze Zahl zwischen 1 und 100 ist,
y eine ganze Zahl zwischen 1 und 12 ist,
y' eine ganze Zahl zwischen 0 und 12 ist,
p 0 oder 1 ist wenn y' ungleich 0 ist, und wenn y' gleich 0 ist dann ist p gleich 0,
y" eine ganze Zahl zwischen 0 und 12 ist wenn p gleich 1 ist, und y" gleich 0 ist, dann ist p gleich 0.

12. Modifiziertes Oligonukleotid nach Anspruch 10, welches folgender Formel entspricht (XIIc): 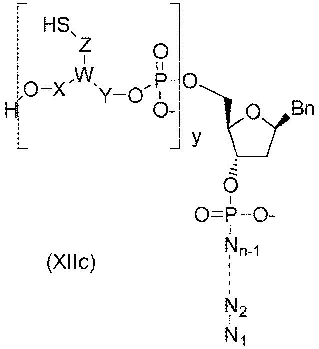 wobei,
n, y, N₁, ... Nₙ₋₁ dieselbe Definition wie in Anspruch 8 haben,
X, Y, Z, W dieselbe Definition wie in den Ansprüchen 1 bis 4 haben,
Bn die Basis des n-ten Nukleotids darstellt.

13. Modifiziertes Oligonukleotid nach Anspruch 11, das folgender Formel (XIIIc) entspricht: 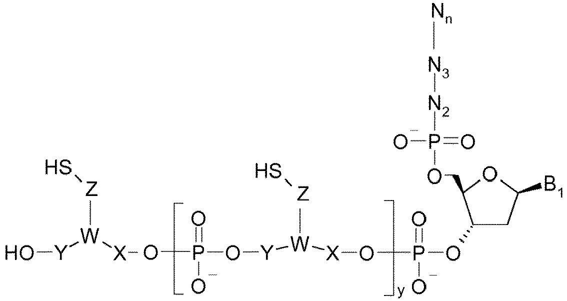 wobei,
n, y, N dieselbe Definition wie in Anspruch 9 haben,
X, Y, Z, W dieselbe Definition wie in den Ansprüchen 1
bis 4 haben,
B₁ die Basis des ersten Nukleotids darstellt.

14. Automatisierte Vorrichtung für die Synthese von Nukleotiden, welche mindestens Folgendes umfasst:
* getrennte Behälter, umfassend:
- Nukleotide,
- Kopplungsaktivatoren und
- Waschprodukte,
* mechanische Mittel zur Entnahme und Verteilung von Produktproben, sowie Informatikmittel, die den kontrollierten Einsatz dieser mechanischen Mittel ermöglichen, sowie:
* mindestens einen Behälter, in dem ein fester Träger angeordnet ist, der mit einem Oligomer nach Anspruch 7 oder 8 gepfropft ist, und/oder mindestens einen Behälter, der eine Verbindung (Ia) oder eine Verbindung (Ib) nach Anspruch 2 enthält.

15. Substrat, das mit mindestens einem modifizierten Oligonukleotid nach einem der Ansprüche 10 bis 13 gepfropft ist, wobei das Substrat mindestens eine Aufnahmezone umfasst, die mit einem Gold- oder Platinfilm überzogen oder mit Gruppierungen gepfropft ist, die mindestens eine Zweifachbindung Kohlenstoff-Kohlenstoff oder eine Dreifachbindung Kohlenstoff-Kohlenstoff oder Halogenacetamid-Gruppierungen, vorzugsweise Maleimid- und/oder Acrylamid-Gruppierungen umfassen, wobei das Substrat aus Metall ist, im Falle des Goldfilms, und aus Kunststoff, im Falle des Pfropfens mit Gruppierungen, die mindestens eine Zweifachbindung Kohlenstoff-Kohlenstoff oder eine Dreifachbindung Kohlenstoff-Kohlenstoff oder Halogenacetamid-Gruppierungen, vorzugsweise Maleimid- und/oder Acrylamid-Gruppierungen umfassen.

16. Substrat nach dem vorhergehenden Anspruch, wobei das Metallsubstrat aus Kupfer oder aus Titan und/oder das Kunststoffsubstrat aus Polystyrol ist.

17. Testbausatz, umfassend:
- mindestens ein Substrat, welches mindestens eine Aufnahmezone umfasst, die mit einem Gold- oder Platinfilm überzogen ist oder mit einem Substrat, das mit Gruppierungen gepfropft ist, die mindestens eine Zweifachbindung Kohlenstoff-Kohlenstoff oder eine Dreifachbindung Kohlenstoff-Kohlenstoff oder Halogenacetamid-Gruppierungen, vorzugsweise Maleimid- und/oder Acrylamid-Gruppierungen umfassen,
- mindestens ein modifiziertes Oligonukleotid nach einem der Ansprüche 10 bis 13.

18. Verwendung eines Substrats nach Anspruch 15 zur Durchführung der Affinitätstests zwischen einem Oligonukleotid und einem anderen Molekül.

## Claims

1. A compound corresponding to the following formula (I): 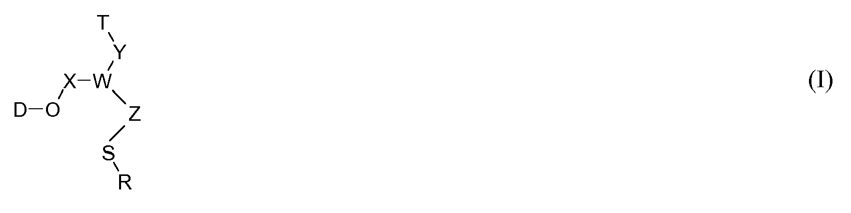 in which:
T is a group selected from -O-P(OR₁)N(R₂)₂, -O-PH(O)O-, -OC(O)JC(O)NH- □,
∘ R₁ is selected from the 2-cyanoethyl, R'₁R'₂R'₃SiCH₂CH₂, groups and R'₁, R'₂, R'₃, which may be identical or different, represent a group selected from the linear or branched alkyls comprising from 1 to 12 carbon atoms and the C6-C12 aryls,
∘ R₂ is selected from the linear or branched alkyl groups comprising from 1 to 12 carbon atoms, pyrrolidine,
∘ J is selected from a single bond, a -CH₂-, -CH₂CH₂-, -CH₂OCH₂-,-CH₂OPhOCH₂- group, where Ph is a benzyl,
∘ □ represents a solid support,
D is a protective group of the alcohols selected from 4,4'-dimethoxytrityl, 9-phenylxanthen-9-yl, fluorenylmethoxycarbonyl and tert-butyl-dimethylsilyl,
W is selected from the CH, CCH₃, CCH₂CH₃, the cyclohexane triyl and the benzene triyl groups,
Z is selected from the C1-C12 alkoxy groups, oxygen-containing or nitrogen-containing C3-C12 cycloheteroalkyl groups, C1-C12 NCO-alkyl groups, C1-C12 CON-alkyl groups,
Y is selected from the linear or branched C1-C12 alkyl groups, C1-C12 aminoalkyl groups, C1-C12 alkoxy groups, C3-C12 cycloalkyl groups, oxygen-containing or nitrogen-containing C3-C12 cycloheteroalkyl groups,
X is selected from the linear or branched C1-C12 alkyl groups, C1-C12 aminoalkyl groups, C1-C12 alkoxy groups, C3-C12 cycloalkyl groups, oxygen-containing or nitrogen-containing C3-C12 cycloheteroalkyl groups,
R is selected from the C1-C12 acyl, C1-C12 S-alkyl, C6-C12 S-aryl, S-2-pyridine, oxygen-containing or nitrogen-containing C1-C12 S-heteroalkyl, C3-C12 S-cycloalkyl, oxygen-containing or nitrogen-containing C3-C12 S-cycloheteroalkyl groups.

2. The compound according to claim 1, corresponding to one of the formulas (Ia), (Ib) and (Ic) below:

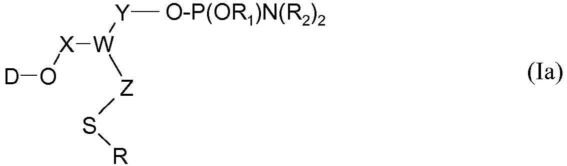

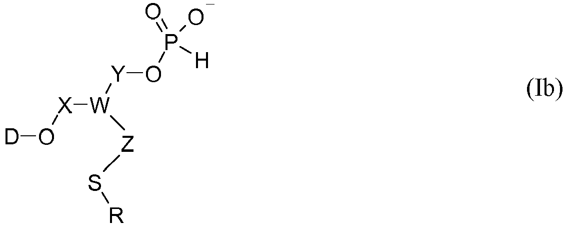

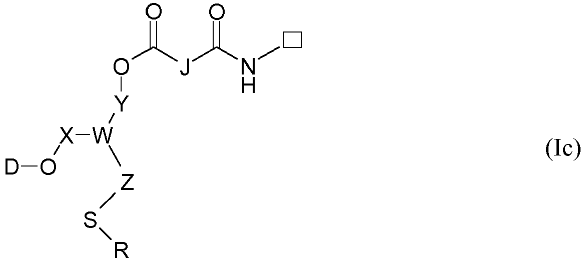

3. The compound according to claim 1 or claim 2, wherein:
• D is selected from 4,4'-dimethoxytrityl, 9-phenylxanthen-9-yl or fluorenylmethoxycarbonyl;
• W is selected from the CH, CCH₃, CCH₂CH₃, the cyclohexane triyl and the benzene triyl groups; and/or
• Z is selected from the C1-C12 alkoxy groups, oxygen-containing or nitrogen-containing C3-C12 cycloheteroalkyl groups, C1-C12 NCO-alkyl groups, C1-C12 CON-alkyl groups; and/or
• Y is selected from the linear or branched C1-C6 alkyl groups, C1-C6 aminoalkyl groups, C1-C6 alkoxy groups, C3-C6 cycloalkyl groups, oxygen-containing or nitrogen-containing C3-C6 cycloheteroalkyl groups; and/or
• X is selected from the linear or branched C1-C6 alkyl groups, C1-C6 aminoalkyl groups, C1-C6 alkoxy groups, C3-C6 cycloalkyl groups, oxygen-containing or nitrogen-containing C3-C6 cycloheteroalkyl groups; and/or
• R is selected from the C1-C6 acyl, C1-C6 S-alkyl, C6-C6 S-aryl, oxygen-containing or nitrogen-containing C1-C6 S-heteroalkyl, C3-C6 S-cycloalkyl, oxygen-containing or nitrogen-containing C3-C6 S-cycloheteroalkyl groups, preferably R is a C1-C6 acyl group.

4. The compound according to any one of claim 1 to 3, wherein T is a group -O-P(OR₁)N(R₂)₂ where R₂ is an isopropyl group and R₁ is chosen from 2-cyanoethyl, 2-(trimethylsilyl)ethyl, 2-(triphenylsilyl)ethyl, 2-(diphenylmethylsilyl)ethyl groups.

5. The compound according to any one of claim 1 to 3, wherein T is a group -OC(O)JC(O)NH-□ where □ is a solid support selected from resins, in particular from resins based on polystyrene, polyacrylamide, polyethyleneglycol, cellulose, polyethylene, polyester, latex, polyamide, polydimethylacrylamide, hydrophilic natural or synthetic polymers, glass beads, silica gels.

6. A process for manufacturing a compound of formula (I) according to any one of claims 1 to 5, comprising at least one step selected from the following steps:
- Preparation of the compound (Ia) starting from compound (II) according to the following synthesis diagram: 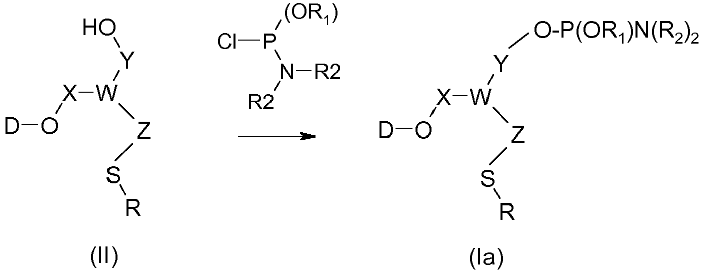 or according to the following synthesis diagram: 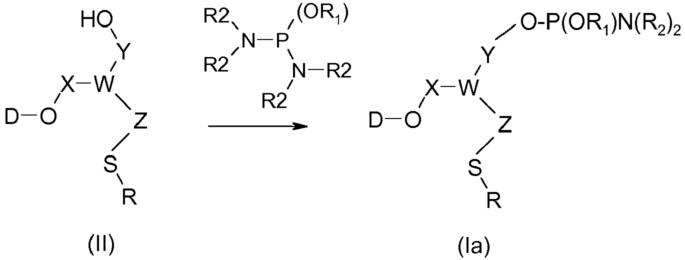
- Preparation of the compound (Ib) starting from compound (II) according to the following synthesis diagram: 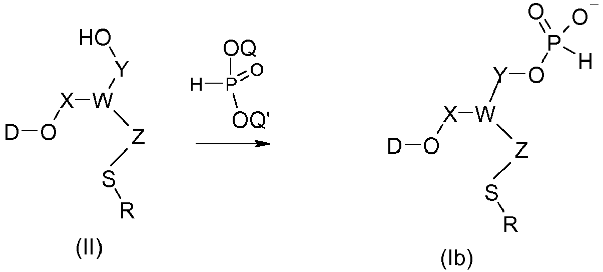 in which Q and Q' represent, independently of one another, a substituted or unsubstituted benzene group,
- Preparation of the compound (Ic) starting from compound (II) according to the following synthesis diagram: 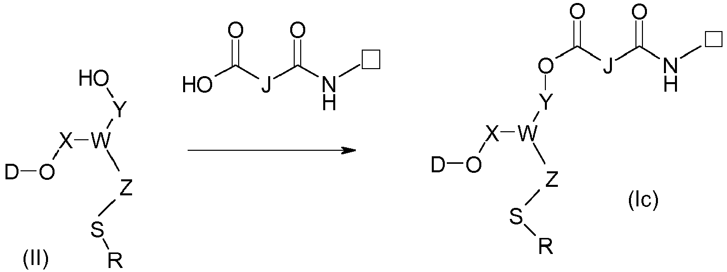 or according to the following synthesis diagram: 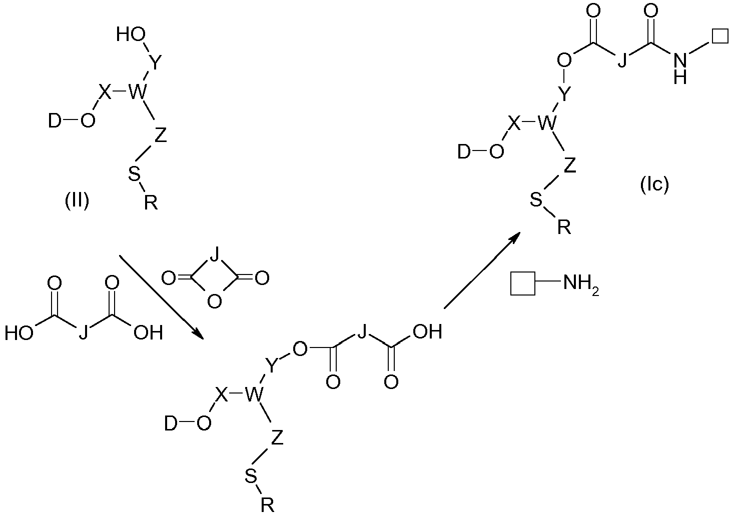 D, X, Y, Z, W, R, □, R1 and R2 having the same definition in each of these steps as in claims 1 to 5.

7. An oligomer obtainable by oligomerization of a compound (I) according to one of claims 1 to 5, having the formula:
(Ic) - (Δ)ₖ (XVI)ₖ
in which:
(Ic) has the same meaning as in claims 2 to 5, R group of compound (Ic) can further represent H,
k represents an integer between 1 and 12,
(Δ) represents (I'a) or (I'b), with: 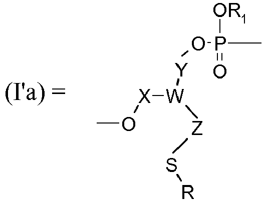 and 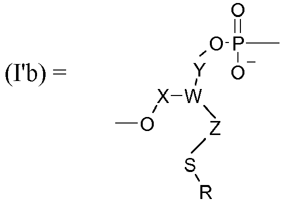
in which X, Y, W, Z, R, 1 have the same definition as in claims 1 to 5, R can further represent H.

8. The oligomer according to claim 7, having the formula (XIV)ₖ: 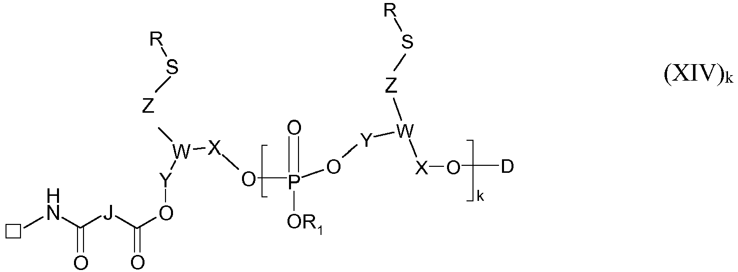 in which:
□, D, X, Y, W, Z, R and R₁ have the same definition as in claims 1 to 4, R can further represent H and D can further represent H,
k is an integer comprised between 1 and 11.

9. A process for manufacturing a modified oligonucleotide comprising at least:
- a step of grafting a compound (I) according to any one of claims 1 to 5 on an oligonucleotide, or
- a step of grafting a nucleotide on an oligomer according to claim 7 or claim 8.

10. An oligonucleotide modified, obtainable by the process according to claim 9, having the following formula (XIIa):
□ - N₁ - N₂- ...- Nₙ₋₁- Nₙ- (I')_{y} -(M₁ -...- Mₘ₋₁- Mₘ)ₚ - (I')_{y'}
in which,
N₁, ...Nₙ represent, independently of one another, a nucleotide,
M₁, ...Mₘ represent, independently of one another, a nucleotide,
(I') represents a compound of formula (I'a) or (I'b) as defined above,
n is an integer comprised between 1 and 100,
m is an integer comprised between 1 and 100,
y is an integer comprised between 1 and 12,
p represents 0 or 1,
y' is an integer comprised between 0 and 12 if p has the value 1, and y' is equal to 0 if p has the value 0,
□ represents a solid support.

11. An oligonucleotide modified, obtainable by the process according to claim 9, having the following formula (XIIIa):
(Ic) - (I')_{y-1}- N₁ - N₂ - ...- Nₙ₋₁- Nₙ - (I')_{y'} -[M₁ - M₂ -... - Mₘ₋₁ - Mₘ]ₚ-(I')_{y"}
in which,
N₁, ...Nₙ represent, independently of one another, a nucleotide,
M₁, ... Mₘ represent, independently of one another, a nucleotide,
(I') represents a compound of formula (I'a) or (I'b) as defined above,
n is an integer comprised between 1 and 100,
m is an integer comprised between 1 and 100,
y is an integer comprised between 1 and 12,
y' is an integer comprised between 0 and 12,
p has the value 0 or 1 if y' is different from 0, and if y' has the value 0 then p has the value 0,
y" is an integer comprised between 0 and 12 if p has the value 1, and if p has the value 0 then y" has the value 0.

12. The modified oligonucleotide according to claim 10, having the formula (XIIc): 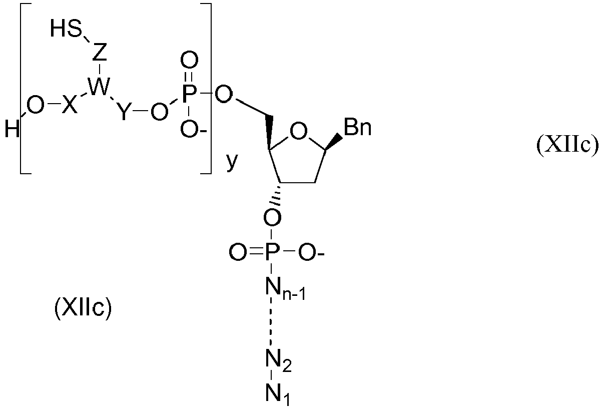 in which,
n, y, N₁, ...Nₙ₋₁, have the same definition as above,
X, Y, Z, W have the same definition as above,
Bn represents the base of the n^{th} nucleotide.

13. The modified oligonucleotide according to claim 11, having the formula (XIIIc): 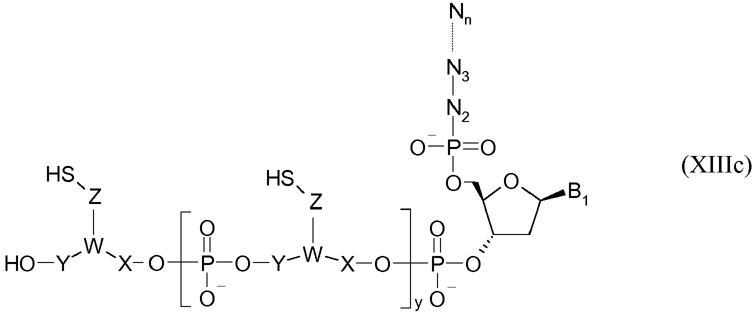 in which,
n, y, N have the same definition as above,
X, Y, Z, W have the same definition as above,
B1 corresponds to the base of the first nucleotide.

14. An automated device for the synthesis of nucleotides comprising at least
distinct containers containing:
- Nucleotides,
- Coupling activators and
- washing products,
mechanical means for sampling and distribution of product samples, as well as computer means for the controlled implementation of these mechanical means, and:
at least one container in which is placed a solid support grafted with an oligomer according to claim 7 or 8, and/or at least one container containing a compound (Ia) or a compound (Ib) according to claim 2.

15. A substrate grafted with at least one modified oligonucleotide according to one of claims 10 to 13, said substrate comprising at least one receiving zone coated with a gold or platinum film or grafted with groups comprising at least one carbon-carbon double bond or a carbon-carbon triple bond or haloacetamide groups, preferably maleimide or acrylamide groups, said substrate being in metal in case of gold film and in plastic in case of grafting with groups comprising at least one carbon-carbon double bond or a carbon-carbon triple bond or haloacetamide groups, preferably maleimide or acrylamide groups.

16. The substrate according to claim 15, wherein the substrate in metal is in copper or in titanium and/or the substrate in plastic is in polystyrene.

17. A test kit comprising:
- at least one substrate comprising at least one receiving zone coated with a gold or platinum metallic film or a substrate grafted with groups comprising at least one carbon-carbon double bond or a carbon-carbon triple bond or haloacetamide groups, preferably maleimide or acrylamide groups,
- at least one modified oligonucleotide according to one of claims 10 to 13.

18. Use of a substrate according to claim 15 to produce affinity tests between an oligonucleotide and another molecule.


### SEQUENCE LISTING

<110> Centre National de la Recherche Scientifique (CNRS) Etablissement FranÃ§ais du Sang (EFS) UniversitÃ© de Montpellier 1 UniversitÃ© Claude Bernard Lyon 1
<120> ComposÃ©s thiol et leur utilisation pour la synthÃ^{··}se d'oligonuclÃ©otides modifiÃ©s
<130> 33336 CNRS
<160> 3
<170> BiSSAP 1.0
<210> 1
   <211> 31
   <212> DNA
   <213> artificial sequences
<220>
   <221> source
   <222> 1..31
   <223> /mol_type="DNA" /note="Amorce VHC sens biotinylÃ©e" /organism="artificial sequences"
<400> 1
   tggggatccc gtatgatacc cgctgctttg a 31
<210> 2
   <211> 30
   <212> DNA
   <213> artificial sequences
<220>
   <221> source
   <222> 1..30
   <223> /mol_type="DNA" /note="Amorce VHC anti-sens" /organism="artificial sequences"
<400> 2
   ggcggaattc ctggtcatag cctccgtgaa 30
<210> 3
   <211> 13
   <212> DNA
   <213> artificial sequences
<220>
   <221> source
   <222> 1..13
   <223> /mol_type="DNA" /note="Acide nuclÃ©ique test greffage" /organism="artificial sequences"
<400> 3
   ccaagcacga tgt 13